(19) Europäisches Patentamt
European Patent Office
Office européen des brevets

(11) EP 1 630 176 A1

(12) EUROPEAN PATENT APPLICATION
published in accordance with Art. 158(3) EPC

(43) Date of publication:
**01.03.2006 Bulletin 2006/09**

(21) Application number: **04731763.1**

(22) Date of filing: **07.05.2004**

(51) Int Cl.:
***C08B 37/00*** *(1974.07)*

(86) International application number:
**PCT/JP2004/006512**

(87) International publication number:
**WO 2004/099258 (18.11.2004 Gazette 2004/47)**

(84) Designated Contracting States:
**AT BE BG CH CY CZ DE DK EE ES FI FR GB GR HU IE IT LI LU MC NL PL PT RO SE SI SK TR**

(30) Priority: **09.05.2003 JP 2003167131**

(71) Applicant: **TOHO CHEMICAL INDUSTRY CO., LTD.
Tokyo 104-0044 (JP)**

(72) Inventors:
- **TAKEDA, Hiromitsu
Sodegaura-shi, Chiba 2990296 (JP)**
- **MORI, Yoshihiko
Sodegaura-shi, Chiba 2990296 (JP)**

(74) Representative: **Schaad, Balass, Menzl & Partner AG
Dufourstrasse 101
Postfach
8034 Zürich (CH)**

# (54) CATION-MODIFIED GALACTOMANNAN POLYSACCHARIDE AND COSMETIC COMPOSITION CONTAINING THE SAME

(57) The present invention provides a cation-modified galactomannan polysaccharide which gives excellent conditioning effects and gives good feeling and flexibility when it is used in a hair treatment compostion, and improves feeling of use by conditioning effects and emulsification conditions when it is used in a skin cosmetic composition such as body cleansing agent.

Concretely, the present invention provides a cation-modified galactomannan polysaccharide obtained by substituting some of hydroxyl groups contained in an cation-modified galactomannan polysaccharide which is nonionic polysaccharide and constitutes mannose as a main chain and galactose as a side chain and the composition ratio of mannose and galactose is 1:1 and obtained obtained from albumen portion of a seed of fenugreek (Trigonella foenum-graecum) which is a leguminus plant, with a specific quaternary nitrogen-containing group, and a cosmetic composition containing the cation-modified cation-modified galactomannan.

EP 1 630 176 A1

## Description

Technical Field

The present invention relates to a cation-modified galactomannan polysaccharide which, when added to a cosmetic composition, produces its adsorption to the hair and skin, thereby enhancing its conditioning effects, and, since the galactomannan polysaccharide has emulsifiability, it helps to exert good finished feel with free from rigidity, starch, etc. after drying, and to such a cosmetic composition containing containing such a cation-modified galactomannan polysaccharide, particularly a hair treatment composition. Background technology

A hair treatment composition particularly directed to hair cleansing contains a conditioning agent for treating damaged hairs which occur during washing or rinsing as a result of the hairs being vigorously agitated and brought into heavy contact with each other, and for improving feeling after washing. To satisfy the desired functions, the conditioning agent must be adsorbed to the surface of hairs. Suitable substances having such conditioning effects mainly include cationic polymers which have ion-based adsorption ability. The cationic polymer may include water-soluble polymers obtained by introducing a quaternary nitrogen-containing group into polysaccharides such as cellulose derivatives, guar gum, starch, etc., and homopolymers of a dialkyldiallyl ammonium salt. For example, Patent Literature 1 discloses the use of a cation-modified cellulose derivative incorporating a quaternary nitrogen-containing group as a constituent of a shampoo or hair cosmetic preparation. In addition, Patent Literature 2 discloses the use of cation-modified starch incorporating a quaternary nitrogen-containing group as a constituent of hair care products such as shampoos, hair rinses, etc.

Furthermore, Patent References 3, 4 and 5 describe cation-modified guar gum by cation-modifying guar gam in which mannose, a main chain, belonging to galactomannan polysaccharide and galactose, a side chain, is 2:1, the guar gam being fenugreek gum of the present invention. Patent Literature 6 describes a cation-modified locust-bean gum by cation-modifying locust-bean gum in which mannose, a main chain, and galactose, a side chain, is 4:1. Patent Literature 7 describes to use cation-polygalactomannan by cation-modifying guar gum as galactomannan polysaccharides and locust-beam gum having the similar structure as hair-care products such as shampoo, rinse, etc. and body soap. In a case of the cation-modified guar gum which uses guar gum, however, the feature of the guar gum is brought out even after the cation-modification, and it is different from the cation-modified galactomannan polysaccharides of the present invention in functions, e.g., emulsifiability, and accordingly, when it is contained in a hair-care composition and skin cosmetic composition, aspect in feeling is different between the cation-modified guar gum and the present invention. Similarly, in a case of cation-modified locust-bean gum which uses locust-bean gum, it is different from the cation-modified galactomannan polysaccharides of the present invention in functions, e.g., emulsifiability, and accordingly, when it is contained in a hair-care composition and skin cosmetic composition, aspect in feeling is different between the cation-modified locust-bean gum and the present invention. Patent Literature 8 describes cation-modified galactomannan polysaccharides obtained by using guar gum, locust-bean gum as galactomannan polysaccharides and decomposing them by enzyme, etc. to be oligomerized and then, subjecting to cation-modification. Patent Literature 9 describes cation-modified galactomannan polysaccharides obtained by using guar gum, locust-bean gum as galactomannan polysaccharides and conducting cation-modification after hydroxyalkyl modification, and further subjected to oligomerization. However, these Patent Literatures and the present invention are different in compositional ratio of main chain and side chain of galactomannan polysaccharide decomposing them by enzyme, etc. to be oligomerized and then, in constitution in which hydroxyalkyl group exists or not, and viscosity caused by the exisistence of a step of making oligomerization. By these differences, these publications are different from the cation-modified galactomannan polysaccharides by cation-modifying galactomannan polysaccharides in which mannose as a main chain and galactose as a side chain is 1:1. Furthermore, the function and emulsifiability as a conditioning agent is naturally different between the present invention and the Patent Literatures from the difference in structure and physical property.

For skin care compositions, soap or an anionic surfactant is generally used as a constituent. However, soap or an anionic surfactant may eliminate such a large amount of the oil and fat component of the skin during washing that the skin may feel taut after washing. To avoid such an inconvenience and to confer moist feeling to the skin, a conditioning agent such as a cationic polymer and a moisture-retaining agent such as glycerin are added even to a skin-cosmetic composition such as a body cleansing agent.

Patent Literature 10 discloses that a shampoo composition to which a copolymer of a dialkyldiallyl ammonium salt and a cellulose derivative incorporating a quaternary nitrogen-containing group provides foamy touch with smooth, slipping finger-passing in hair washing and supplies hair with dry smoothness and easy finger passing in rinsing oily coats on the hairs, and excellent luster in finish. Such a cationic polymer is also used as a constituent of skin care compositions such as body cleansers, because the resulting composition can develop creamy foam quality, and the skin washed with the composition is free from the tautness after drying which would otherwise occur, and has moist feeling.

On the other hand, with respect to fenugreek gum which is galactomannan polysaccharide in which composition ratio of mannose as a main chain and galactose as a side chain is 1:1, which is obtained from rom albumen portion of seed of fenugreek (*Trigonella foenum-graecum*) which is a leguminus plant, Patent Literatures 11 and 12 describe

fenugreek which is a raw material of the fengugreek gum, as a raw material of a spice. However, these Patent Literatures do not describe that the cation-modified galactomannan polysaccharides of the present invention prepared by introducing quaternary nitrogen-containing group into fenugreek gum obtained from albumen portion of seed of fenugreek exerts excellent effects as conditioning agents.

Patent Literature 1: Japanese Patent Publication No. Sho 47-20635 (page 5)
Patent Literature 2: Japanese Patent Publication No. Sho 60-42761 (pages 1 to 9)
Patent Literature 3: Japanese Patent Application Laid-open No. Sho 55-164300 (pages 1 to 2)
Patent Literature 4: Japanese Patent Application Laid-open No. Hei 4-364111 (pages 1 to 6)
Patent Literature 5: Japanese Patent Publication No. Hei 7-17491 (pages 1 to 4)
Patent Literature 6: Japanese Patent Application Laid-open No. 2000-103724 (pages 1 to 6)
Patent Literature 7: Japanese Patent Publication No. Hei 7-238186 (pages 2 to 3, pages 5 to 6)
Patent Literature 8: Japanese Patent Application Laid-open No. Hei 7-173029 (pages 1 to 7)
Patent Literature 9: Japanese Patent No. 3349219 (pages 1 to 6)
Patent Literature 10: Japanese Patent Application Laid-open No. Hei 1-128914 (pages 1 to 3)
Patent Literature 11: Japanese Patent Application Laid-open No. Sho 62-134061 (page 1)
Patent Literature 12: Japanese Patent Application Laid-open No. Hei 10-215808 (page 2)

When a cation-modified cellulose derivative is added to a hair treatment composition, and the composition is applied to hair, the cellulose derivative reacts with an anionic surfactant to form a conjugated salt which is adsorbed to hair so readily that it exerts an excellent conditioning effect during rinsing. However, after drying, rigidity appears in hair and feel of hair beomces worse. If cation-modified guar gum is used for the same purpose, the rigidity of hair after drying is less, but its adsorption to the hairs is so low that its conditioning effect is less notable during rinsing. Moreover, such a problem is caused that by the recent multiplicity of fashion, there are amny chances to use hair color and hair dye, and according to the use thereof, hair is remakably damaged; and even though cation-modified guar gum and cation-modified locust-bean gum are used, flexibility is obliged to lack in the damaged hair so that rigidity is present in the damaged hair. If a conditioning agent such as a cationic polymer or a moisture-retaining agent such as glycerine is added to a skin cosmetic composition, the composition will become so sticky or slimy depending on the amounts added.

Based on the situations described above as a background, the present inventors studied hard to seek a compound which, when added to a hair treatment composition, in contrast with conventional cationic polymers, has an improved conditioning effect during rinsing, and ensures an improved finished feel of the hair after drying, and when added to a skin cosmetic composition, makes the skin devoid of tautness and chapping, and improves stickiness and sliminess. As a result of the study, they found that this object was met by a cation-modified galactomannan polysaccharide obtained by introducing a specific amount of quaternary nitrogen-containing group into a nonionic polysaccharide and adjusting the cation charge density of the resulting compound within a specified range. The galactomannan polysaccharide is kind of galactomannan polysaccharides which constitute mannose as a main chain and galactose as a side chain, and the composition ratio of mannose and galactose is 1:1. Namely, the cation-modified galactomannan polysaccharide has an excellent property as a coditioning agent in a cosmetic composition. Moreover, since the cation-modified galactomannan polysaccharide has emulsifiability, appropriate oil moiety (fat and oil component) remains in hair or skin, thereby improving flexibility and feel can be improved. This finding led them to the present invention.

Disclosure of the invention

The present invention provides a cation-modified galactomannan polysaccharide which is suitably used as a constituent of various hair treatment compositions such as shampoos, hair rinses, hair colors, etc.; skin cosmetic compositions; and cosmetic compositions such as make-up agents. The cation-modified galactomannan polysaccharide is a cation-modified polysaccharide obtained by substituting some of hydroxyl groups contained in a galactomannan polysaccharide which constitutes mannann as a main chain and galactose as a side chain, and the composition ratio of mannose and galactose is 1:1, with quaternary nitrogen-containing groups of the following Formula (1), in which the cationic charge density due to the quaternary nitrogen-containing group is adjusted to be in the range of 0.1 to 3.0 meq/g:

[Chemical Formula 1]

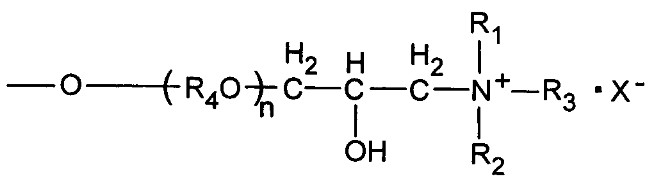

where $R_1$ and $R_2$ independently represent alkyl groups having 1-3 carbon atoms; $R_3$ is an alkyl group having 1-24 carbon atoms; X- is an anion; and n is n=0 or n=1-30, and when n=1-30, $(R_4O)_n$ represents a residue of an alkyleneoxide polymer having 2-4 carbon atoms, that is, a polyalkylene glycol chain comprising a unialkyleneoxide and/or two or more different alkyleneoxides.

Best mode to carry out the invention

The galactomannan polysaccharides used in the present invention is nonionic polysaccharides which constitute mannose as a main chain and galactose as a side chain and the composition ratio of the mannose and the galactose is 1:1, and is a natural water-soluble gum obtained from albumen portion of seed of fenugreek (*Trigonella foenum-graecum*) which is a leguminus plant which is an annual herb cultivated in the Mediterranean area as the place of origin, the Middle and Near East, Africa and India. The natural water-soluble gum is generally called as fenugreek gum which is easily obtained as "Fenugreek Albumen Powder A" and as "Fenugreek Albumen Powder B" (manufactured by Air Green Co., Ltd.).

It is well-known fact that the fenugreek gum which is galactomannan polysaccharides in which the composition ratio of the mannose and the galactose is 1:1 is different from the the similar galactomannan polysaccharide, e.g., guar gum in which a composition ratio of mannose and galactose is 2:1 and from locust-bean gum in which a composition ratio of mannose and galactose is 4:1, in rheology of an aqueous solution. Moreover, emulsifiability of the galactomannan polysaccharides used in the present invention is good in comparison with guar gum or locust-bean gum, due to the composition ratio and the rheology of solution.

Cation-modification according to the method of the present invention can be achieved by allowing a galactomannan polysaccharide in which the composition ratio of mannose and galactose is 1:1 (hereinafter referred to as "fenugreek gum") to react with glycidyltrialkyl ammonium salt or 3-halogeno-2-hydroxypropyltrialkyl ammonium salt, both of which contain a quaternary nitrogen-containing group. The reaction is preferably allowed to occur in an appropriately chosen solvent, preferably in hydrous alcohol, in the presence of alkali. Introduction of such a quaternary nitrogen-containing group may be achieved by any known methods, but is not necessarily limited to those methods. For example, it is possible to obtain a cation-modified galactomannan polysaccharide, i.e., cation-modified fenugreek gum, of the present invention by adding alkyleneoxide having 2-4 carbon atoms to some of hydroxyl groups contained in a galactomannan polysaccharide in which the composition ratio of mannose and galactose is 1:1, and then allowing the mixture to react with a quaternary nitrogen-containing group of either glycidyltrialkyl ammonium salt or 3-halogeno-2-hydroxypropyltrialkyl ammonium salt mentioned above. An inorganic salt, preferably sodium chloride, may be added to the reaction system to prevent the aggregation of fenugreek gum in the solvent at the reaction time. In order to prevent the aggregation of fenugreek gum, enhance the dispersion of solutes, and increase the yield, an alkali or inorganic salt is preferably added and dissolved or dispersed in the solvent, and then the fenugreek gum is added and dissolved or dispersed, which is followed by the addition of an aforementioned the quaternary nitrogen-containing group.

With regard to a quaternary nitrogen-containing group to be introduced into the fenugreek gum whose structure is as shown in the chemical Formula (1), examples of $R_1$ and $R_2$ include methyl, ethyl and propyl groups. Examples of $R_3$ as alkyl group having 1-24 carbon atoms include octyl, decyl, dodecyl, tetradecyl, hexadecyl, octadecyl and docosyl groups, in addition to those mentioned above with respect to $R_1$ and $R_2$. Examples of $R_4O$ include ethoxy, propoxy and butoxy groups. Examples of X- include, in addition to halogen ions such as chlorine ions, bromine ions and iodine ions, methyl sulfate ions, ethyl sulfate ions and acetate ions.

The cation-modified galactomannan polysaccharide (hereiafter referred to as "cation-modified fenugreek gum") of the present invention has emulsifiability and adsorbability to hair and skin and thus, it is excellent as blending component of a cosmetic composition. For example, when it is added to a hair care composition, conditioning effect and flexibility are given. If it is added to a skin cosmetic composition, e.g., a body cleanser, it does not eliminate more amount of the fat and oil component of the skin than need, and improves the texture of the skin. Accordingly, the present invention

also relates to a cosmetic composition to which the cation-modified galactomannan polysaccharide of the present invention is added.

The cation charge density due to quaternary nitrogen-containing group of the cation-modified galactomannan polysaccharide of the present invention is 0.1 to 3.0 meq/g, preferably 0.5 to 2.5 meq/g. If the cation charge density is below 0.1 meq/g, the adsorption of the cation-modified galactomannan polysaccharide to the hair would be so low that, even if it is added to a hair treatment composition such as shampoo, hair rinse or body cleanser, or to a skin cosmetic composition, the composition would not exhibit the expected effects. On the contrary, if the cation charge density were beyond 3.0 meq/g, even if such a cation-modified galactomannan polysaccharide compound is added to a hair treatment composition or skin cosmetic composition, in use, lathering would be impaired, the hair would become so sticky and slimy as to make worse feel in use, and finished feel after would be disagreeably rigidity and sticky.

The cation charge density due to the cationic nitrogen-containing group of the cation-modified galactomannan polysaccharide refers to equivalent amount of the quaternary nitrogen-containing group whose structure is as shown in Formula (1) contained in 1 g of the cation-modified galactomannan polysaccharide. Normally, the amount in question is obtained by measuring the amount of nitrogen contained in the quaternary nitrogen-containing group using the Kjeldahl method (Standards of Materials of Cosmetic Products, previous edition, General Testing Methods, Quantification of nitrogen content, 2nd method), and determining the sought amount based on the measurement result by calculation. However, a galactomannan polysaccharide in which the composition ratio of galactose and mannose is 1:1 used in the present invention contains a nitrogen moiety. Therefore, the sought amount of nitrogen contained in a quaternary nitrogen-containing group is obtained by subtracting the amount of nitrogen due to a galactomannan polysaccharide in which composition ratio of galasctose and mannose is 1:1 used as a starting material of the present invention from the amount of nitrogen determined by the Kjeldahl method for a cation-modified galactomannan polysaccharide of the present invention. Concretely, in a case that nitrogen amount of the present invention obtained by cation-modifying sodium alginate having a quaternary nitrogen-containing group of Formula (1) wherein $R_1$, $R_2$ and $R_3$ represent methyl groups, X- is a chloride ion, and n is n=0, is 1.60 % by the measuring result of the Kjeldahl method, cation charge density of the substance can be obtained from following equation. Nitrogen at about 0.40% is normally contained in fenugreek gum used by the invention.

Cation charge density (meq/g) = (nitrogen amount (%) due to

quaternary nitrogen-containing group x 1000)/(atomic weight of nitrogen

= 14.0) x 100 = (1.60 - 0.40)/1.40

= 0.86

The cation-modified galactomannan polysaccharide of the invention is added to a hair treatment composition or a skin cosmetic composition preferably at 0.05-5% by weight with respect to the weight (100% by weight) of the entire composition. If the amount added were below 0.05% by weight, the resulting composition would have tendency to sufficiently exert conditioning effect and emulsifiability. On the contrary, if the amount added were beyond 5% by weight, the resulting composition would have tendency to make the hair or skin sticky and slimy, impair its flexibility, and make the feel in use worse.

To a hair treatment composition of the present invention, various kinds of cationic water-soluble polymers and amphoteric water-soluble polymers may be added, in addition to a cation-modified galactomannan polysaccharide, in order to further enhance the conditioning effect of the composition. However, the amount of the additional polymer added should be adjusted so as not to interfere with the emulsifiability and flexibility to hair of a cation-modified galactomannan polysaccharide used in combination. The amount in question added is preferably 5% by weight or lower, with respect to the weight (100% by weight) of the entire composition. If the amount added exceeded the above range, rigidity is generated in use, and not only emulsifiability produced by cation-modified galactomannan polysaccharide becomes not to be sufficiently exerted, but also there is tendency that flexibility to hair is worsened. Moreover, if additional polymer is added to a skin cosmetic composition, sliminess is generated and feeling in use become worse.

Suitable cationic water-soluble polymers and amphoteric water-soluble polymers include the following compounds, but they are not necessarily limited to these compounds.

The cationic water-soluble polymer may include a quaternary nitrogen-based cationic polysaccharide (cation-modified hydroxyethyl cellulose, cation-modified guar gum, cation-modified locust-bean gum, cation-modified starch, cation-modified tara gum, cation-modified tamarind gum, etc.), dimethyldiallyl ammonium chloride derivatives (copolymers of dimethyldiallyl ammonium chloride and acrylamide, polychlorinated dimethylmethylenepiperidinium, etc.), vinylpyrrolidone derivatives (copolymers of vinylpyrrolidone and dimethylaminoethylmethacrylate, copolymers of vinylpyrrolidone and methacrylamidopropyltrimethylammonium chloride, copolymers of vinylpyrrolidone and methylvinylimidazolium chloride, etc.), and methacrylate derivatives (copolymers of methacryloylethyldimethylbetaine, methacryloylethyltrimethylammonium chloride and methacrylic acid 2-hydroxyethyl, copolymers of methacryloylethyldimethylbetaine, methacryloylethyltrimethylammonium chloride and methacrylic acid methoxypolyethylene glycol, etc.).

The amphoteric water-soluble polymer may include amphoterized starch, dimethyldiallylammonium chloride derivatives (copolymers of acrylamide, acrylic acid and dimethyldiallylammonium chloride, copolymers of acrylic acid and dimethyldiallylammonium chloride, etc.), and methacrylate derivatives (copolymers of polymethacryloylethyldimethylbetaine, N-methacryloyloxyethyl N,N-dimethylammonium-α-methylcarboxybetaine and alkyl methacrylate, etc.)

A cosmetic preparation according to the present invention can be obtained by adding a cation-modified galactomannan polysaccharide of the present invention to a prescribed system at a specified amount by a commonly known method, and other components to be used in combination are not limited to specific ones, but any components generally used in such cosmetic preparations may be used. Such additives to be used in combination include followings.

The anionic surfactant may include alkyl (8-24 carbon atoms) sulfate, alkyl (8-24 carbon atoms) ether sulfate, alkyl (8-24 carbon atoms) benzene sulfonate, alkyl (8-24 carbon atoms) phosphate, polyoxyalkylenealkyl (8-24 carbon atoms) ether phosphate, alkyl (C 8-24) sulfosuccinate, polyoxyalkylenealkyl (8-24 carbon atoms) ether sulfosuccinate, acyl (8-24 carbon atoms) alaninate, acylatd (8-24 carbon atoms) N-methyl-β-alaninate, acylated (8-24 carbon atoms) glutamate, acylated (8-24 carbon atoms) isethionate, acylated (8-24 carbon atoms) sarcosinate, acylated (8-24 carbon atoms) taurinate, acylated (8-24 carbon atoms) methyl taurinate, α-sulfofatty acid ester salt, ether carbonate, polyoxyalkylene fatty acid monoethanol amide sulfonate, long chain (8-24 carbon atoms) carboxylate, etc.

The nonionic surfactant may include alkanol amide, glycerin fatty acid ester, polyoxyalkylenealkyl ether, polyoxyalkyleneglycol ether, polyoxyalkylenesorbitan fatty acid ester, sorbitan fatty acid ester, polyoxyalkylenesorbit fatty acid ester, sorbit fatty acid ester, polyoxyalkyleneglycerin fatty acid ester, polyoxyalkylene fatty acid ester, polyoxyalkylenealkylphenyl ether, tetrapolyoxyalkylene ethylenediamine-condensed substances, sucrose fatty acid ester, polyoxyalkylene fatty acid amide, polyoxyalkyleneglycol fatty acid ester, polyoxyalkylene castor oil derivatives, polyoxyalkylene-hardened castor oil derivatives, alkylpolyglycoside, polyglycerin fatty acid ester, etc.

The amphoteric surfactant may include alkyl (8-24 carbon atoms) amidopropylbetaine, alkyl (8-24 carbon atoms) carboxybetaine, alkyl (8-24 carbon atoms) sulfobetaine, alkyl (8-24 carbon atoms) sulfobetaine, alkyl (8-24 carbon atoms) hydroxysulfobetaine, alkyl (8-24 carbon atoms) amidopropylhydroxysulfobetaine, alkyl (8-24 carbon atoms) hydroxyphosphobetaine, alkyl (8-24 carbon atoms) aminocarboxylate, alkyl (8-24 carbons) imidazoliumbetaine, alkyl (8-24 carbon atoms) amineoxide, alkyl (8-24 carbon atoms) phosphate esters containing a tertiary or quaternary nitrogen group, etc.

The oil component may include olive oil, jojoba oil, liquid paraffin, fatty acid alkyl ester, etc. The pearl agent may include fatty acid ethyleneglycol, etc. The suspension may include polystyrene emulsion, etc.

In addition to the cationic or amphoteric water-soluble polymers described above, an anionic or nonionic polymer may be added to a hair treatment composition or skin cosmetic composition of the present invention in order to adjust the viscosity of the composition or improve the performance of the composition in maintaining the finished style, so long as the addition of the polymer does not interfere with the expected effects of the composition of the present invention.

Such anionic polymers may include acrylate derivatives (polyacrylic acid and its salts, and copolymers of acrylic acid, acryl amide and ethyl acrylate, and their salts), methacrylate derivatives (polymethacrylic acid and its salts, copolymers of methacrylic acid, acryl amide, diacetone acrylamide, alkylesteracrylate and alkylestermethacrylate and their salts), crotonate derivatives (copolymers of vinylacetate and crotonic acid, etc.), maleate derivatives (copolymers of anhydrous maleic acid and diisobutylene, copolymers of isobutylene and maleic acid, etc.), polyglutamic acid and its salts, hyaluronic acid and its salts, carboxymethyl cellulose, carboxyvinyl polymer, etc.

The nonionic polymer may include acrylate derivatives (copolymers of hydroxyethyl acrylate and methoxyethyl acrylate, amide polyacrylate, etc.), vinylpyrrolidone derivatives (polyvinylpyrrolidone, copolymers of vinylpyrrolidone and vinyl acetate, etc.), polyoxyalkylene glycol derivatives (polyethylene glycol, polypropylene glycol, etc.), cellulose derivatives (methyl cellulose, hydroxyethyl cellulose, hydroxypropyl cellulose, etc.), polysaccharides and their derivatives (guar gum, locust-bean gum, dextran, etc.), etc.

In another embodiment to a hair treatment composition or skin cosmetic composition of the present invention, an amideamine compound and a neutralizing agent such as an organic and/or inorganic acid may be added together with a higher fatty acid and/or higher alcohol, so as to enhance the conditioning effect of the composition. The amount of an amideamine compound added is preferably 5% by weight or lower with respect to the weight (100% by weight) of the entire composition. If the amount in question added exceeded the above range, feel after use of the resulting composition would become so mat, and sliminess is generated and feel in use become worse.

Other suitable components to be added to a hair treatment composition or to a skin care composition of the present invention may include cationic surfactants (alkyltrimethyl ammonium salts, dialkyldimethyl ammonium salts,

alkylpyridium salts, alkyldimethylbenzyl ammonium salts, benzethonium chloride, benzalkonium chloride, etc.), solubilizers (ethanol, ethylene glycol, propylene glycol, etc.), waxes (carnauba wax, candelilla wax, etc.), hydrocarbon oils (liquid paraffin, squalane, etc.), moisture retainers (glycerin, trehalose, sorbitol, maltitol, dipropylene glycol, 1,3-butylene glycol, sodium hyaluronate, etc.), esters (hexyl laurate, isopropyl myristate, octyldodecyl myristate, myristic acid myristate, myristic acid-2-hexyldecyl, glycerin trimyristate, isopropyl palmitate, palmitic acid-2-heptylundecyl, palmitic acid-2-hexyldecyl, butyl stearate, isocetyl stearate, 12-hydroxystearic acid cholesteryl, cetostearylalcohol, cetyl octanate, hexyldecyldimethyl octanate, isocetyl isostearate, trimethylolpropane triisostearate, decyl oleate, oleic acid oil, cetyl lactate, myristyl lactate, ethyl acetate, butyl acetate/amyl acetate, acetic acid lanolin, 2-ethylhexanoic acid cetyl, 2-ethylhexyl palmitate, di-2-ethylhexylic acid ethylene glycol, tri-2-ethylhexylic acid trimethylol propane, tri-2-ethylhexylic acid glycerin, tetra-2-ethylhexylic acid pentaerythritol, cetyl-2-ethyl hexanoate, diisobutyl adipate, adipic acid-2-heptylundecyl, adipic acid-2-hexyldecyl, dipentaerythritol fatty acid ester, neopentyl glycol dicaprylate, diisostearyl malate, di-2-heptylundecanoic acid glycerin, tri-2-heptylundecanoic acid glyceride, caster oil fatty acid methyl ester, acetoglyceride, N-lauryl-L-glutamic acid-2-octyldodecyl ester, di-2-ethylhexyl sebacate, diisopropyl sebacate, succinic acid-2-ethylhexyl, triethyl citrate, ethyl laurate, mink oil fatty acid ester, etc.), antioxidants (tocopherol, BHT, etc.), silicones (methylpolysiloxane, methylphenylpolysiloxane, high polymeric methylpolysiloxane, cyclic polysiloxane, etc.) and silicone derivatives (polyether-modified silicone, amino-modified silicone, etc.), higher alcohols, higher fatty acids (lauric acid, myristic acid, palmitic acid, stearic acid, behenic acid, isostearic acid, oleic acid, undecylenic acid, tall oil fatty acid, coconut oil fatty acid, palm oil fatty acid, palm kernel fatty acid, linolic acid, linoleic acid, eicosapentaenoic acid, docosahexanoic acid, etc.), amino acids (alginin, glutamic acid, etc.), UV absorbants (benzophenon derivatives, paraamino benzoate derivatives, methoxy cinnamate derivatives, etc.), UV-scattering agents (inorganic compounds such as zinc oxide, zirconium oxide, titanium oxide, etc.), thickeners, metal chelating agents (edetic acid salts, etc.), pH-adjusting agents, bactericides, preserving agents, hair growth stimulants, vitamins, anti-inflammatory agents, dyes, pigments (inorganic white pigments such as titanium dioxide, inorganic red pigments such as iron oxide (Indian red), iron titanate, etc., inorganic green pigments such as cobalt titanate, etc., iron oxide-treated mica titanium, carbon black-treated mica titanium, etc.), perfumes, foaming-promoting agents, etc.

A cosmetic composition prepared according to the present invention as described above is not limited in prescription to any specific one, but may take any desired prescription. Furthermore, any appropriate additive usually used in a common cosmetic composition other than those derived above may be added to a cosmetic composition of the present invention, so long as it does not interfere with the effects originally expected from the composition, and the resulting composition may be processed by conventional techniques into appropriate products. Particularly, such an additive may be added with profit to a hair treatment composition of the present invention. Suitable prescriptions a hair treatment composition of the present invention can take include shampoos, hair rinses, hair conditioners, hair waxes, hair lotions, hair mists, etc. All these prescriptions are based on the features of a cation-modified galactomannan polysaccharide of the present invention added to the composition, that is, its excellent conditioning effects and emulsifiability. Since the cation-modified galactomannan polysaccharide of the present invention, when applied to the skin, has effect to improve feel in use, it is possible to be as a body cleanser, facial cleanser or lotion. Furthermore, it may be added to an acidic hair dye, oxidizing hair dye, perm-setting agent, etc.

## EXAMPLES

Other The present invention will be detailed below by means of the preferred embodiments. However, the present invention is not limited to those embodiments. In the following description, the content of constituents in compositions expressed by "wt%" and "vol%" mean "percent by weight" and "percent by volume", respectively unless otherwise specified.

[Manufacture of a cation-modified galactomannan polysaccharide]

### Example 1

Other 11.8 g of an aqueous 48 wt% sodium hydroxide solution and 3.0 g of sodium chloride were added to 900 ml of an aqueous 80 vol% isopropanol solution. 162 g of fenugreek gum (Fenugreek Albumen Powder A manufactured by Air Green Co., Ltd.) was gradually added and dispersed to the mixture. 40.5 g of an aqueous 80 wt% glycidyltrimethyl ammonium chloride (GTA) solution was added to the mixture, and heated and reacted at 50°C for three hours. After completion of the reaction, the reaction mixture was neutralized with 14.0 g of 35% hydrochloric acid which was diluted with 1500 ml of an aqueous 70 vol% isopropanol solution. After neutralizing at room temperature for one hour, the reaction solution was poured into 800 ml of methanol, and reaction product was precipitated and separated by filtration. The obtained precipitate was washed with an aqueous methanol solution, and the reaction product was dried under reduced pressure. Cationic charge density of the cation-modified galactomannan polysaccharide thus obtained was

0.75 meq/g. The result is shown in Table 1 (under Sample 1 in Table 1).

Other Similarly, cation-modified galactomannan polysaccharides having different cationic charge density were synthesized by changing the amount of GTA to be added. The result is shown in Table 1 (under Samples 2 and 3 in Table 1).

Example 2

Other 162 g of fenugreek gum (Fenugreek Albumen Powder A manufactured by Air Green Co., Ltd.) was dispersed in 900 ml of an aqueous 55 vol% isopropanol solution and added with 6.0 g of sodium chloride and 48.9 g of an aqueous 48 wt% sodium hydroxide solution. Then, 152.3 g of 3-halogeno-2-hydroxypropyldimethylmonolauryl ammonium chloride was added to the mixture, heated and reacted at 50°C for three hours. After completion of the reaction, the reaction mixture was neutralized with 14.0 g of 35% hydrochloric acid which was diluted with 1500 ml of an aqueous 70 vol% isopropanol solution. After neutralizing at room temperature for one hour, the reaction solution was poured into 800 ml of methanol, and reaction product was precipitated and separated by filtration. The obtained precipitate was washed with an aqueous methanol solution, and the reaction product was dried under reduced pressure. Cationic charge density of the cation-modified galactomannan polysaccharide thus obtained was 0.80 meq/g. The result is shown in Table 1 (under Sample 4 in Table 1).

Example 3

Other In an autoclave, 162 g of fenugreek gum (Fenugreek Albumen Powder A manufactured by Air Green Co., Ltd.) was dispersed in 900 ml of an aqueous 80 vol% isopropanol solution and added with 11.8 g of an aqueous 48 wt% sodium hydroxide solution. 66 g of ethylene oxide and 240 g of propylene oxide were added to the mixture and heated and reacted at 70°C for three hours under pressurization and sealing. After completion of the reaction, pressurization was released and the reaction mixture was cooled to 50°C. After cooling, 150 g of an aqueous 80 wt% GTA solution was added to the reaction mixture and reacted at 50°C for three hours. After completion of the reaction, the reaction mixture was neutralized with 14.0 g of 35% hydrochloric acid which was diluted with 1500 ml of an aqueous 70 vol% isopropanol solution. After neutralizing at room temperature for one hour, the reaction solution was poured into 800 ml of methanol, and reaction product was precipitated and separated by filtration. The obtained precipitate was washed with an aqueous methanol solution, and the reaction product was dried under reduced pressure. Cationic charge density of the cation-modified galactomannan polysaccharide thus obtained was 0.84 meq/g. The result is shown in Table 1 (under Sample 5 in Table 1).

Supplement 1

Other The same experimental procedures were repeated except that the added amount of GTA was varied, to produce some additional cation-modified fenugreek gum having different cationic charge densities. These results are also shown in Table 1 (under Samples 6 and 7).

Comparative Example 1

Other For the comparison with the cation-modified galactomannan polysacchardie of the present invention, guar gum (in which the composition ratio of mannose and galactose is 2:1) and locust-bean gum (in which the composition ratio of mannose and galactose is 4:1) were cation-modified according to the method of Example 1. The nitrogen content of the obtained cation-modified guar gum is 1.7% by weight, and cationic charge density was 0.74 meq/g. On the other hand, the nitrogen content of the obtained cation-modified locust-bean gum was 1.9% by weight, and cationic charge density was 0.72 meq/g. The resut is shown in Table 1 (Sampels 8 and 9 in Table 1). Nitrogen contained in unmodified or intact guar gum and locust-bean gum were 0.7& by weight and 0.9% by weight, respectively.

TABLE 1. Cation Charge Density

| Sample No. | Charge density (meq/g) | Structure of galactomannan polysaccharide (composition ratio of mannose and galactose) | Synthesized |
|---|---|---|---|
| 1 | 0.75 | 1 : 1 | in Example 1 |
| 2 | 1.52 | 1 : 1 | in Example 1 |
| 3 | 2.89 | 1 : 1 | in Example 1 |
| 4 | 0.80 | 1 : 1 | in Example 2 |

Table continued

| Sample No. | Charge density (meq/g) | Structure of galactomannan polysaccharide (composition ratio of mannose and galactose) | Synthesized |
|---|---|---|---|
| 5 | 0.84 | 1 : 1 | in Example 3 |
| 6 | 0.06 | 1 : 1 | in Supplement 1 |
| 7 | 4.01 | 1 : 1 | in Supplement 1 |
| 8 | 0.74 | 2 : 1 | in Comparative Example 1 |
| 9 | 0.72 | 4 : 1 | in Comparative Example 1 |

[Production of Cosmetic Preparations Added with Cation-modified Galactomannan Polysaccharides, and their Evaluation]

Example 4. Enhancement of the flexibility of hairs (Part 1)

Other Enhancement of the flexibility of hairs due to a cation-modified galactomannan polysaccharide was evaluated by means of a water-rinse hair cleansing preparation (hair shampoo) supplemented with the cation-modified galactomannan polysaccharide.

(Preparation of hair shampoo)

4-a

The cation-modified galactomannan polysaccharides prepared in Examples 1, 2 and 3 were used to prepare shampoo samples whose composition is as shown in column (A) of Table 2. The shampoo sample was prepared as follows. Ingredient (12) in column (A) of Table 2 was heated to 60°C to which ingredient (1) was slowly added with stirring and the mixture was stirred until it was dissolved. After recognizing the dissolution, heating was discontinued, and ingredient (5), ingredient (6) and ingredient (7) were added to the mixture with stirring at the weight ratios specified in column (A) of Table 2. The mixture was stirred until it became a uniform liquid, to which ingredient (8), ingredient (10) and ingredient (11) were added at the weight ratios specified in Table 2 at 30 to 40°C, and the mixture was stirred to homogeneity. Thus, four kinds of shampoo samples with compositions as shown in column (A) of Table 2 were obtained. The four shampoo samples prepared from the cation-modified galactomannan polysaccharides represented by test samples 1, 3, 4 and 5 in Table 1 were named test prescriptions S1, S2, S3 and S4, respectively, of the present invention.

4-b

Shampoo having composition sown in column (B) of Table 2 was prepared by using the cation-modified prepared galactomannan polysaccharide in Example 1 and named test sample 1 and by further containing a cation-modified hydroxyethyl cellulose containing nitrogen at 1.8 wt% to which an average of 1.8 mol of ethylene oxide was introduced (Catinal HC-100, Toho Chemical Industry Co., Ltd.). Ingredient (12) in column (B) of Table 2 was heated to 60°C to which ingredient (1) and ingredient (3) were slowly added with stirring and were dissolved. After recognizing the dissolution, heating was discontinued, and ingredients (5) to (7) were added to the mixture with stirring. The mixture was stirred until it became a uniform liquid. Ingredients (8) to (11) were added to the mixture at 30 to 40°C, and the mixture was stirred to homogeneity to prepare a shampoo sample. The obtained shampoo sample was named a test prescription S5 of the present invention.

4-c

Shampoo having composition sown in column (B) of Table 2 was prepared by using the cation-modified galactomannan polysaccharide prepared in Example 1 and named test sample 1 and by further containing a copolymer of N-methacryloyloxyethyl N,N-dimethyl ammonium-$\alpha$-methylcarboxylbetaine and alkyl methacrylate (Yukaformer SM, Mitsubishi Chemicals Co.) as an amphoteric water-soluble polymer. Ingredient (12) in column (C) of Table 2 was heated to 60°C to which ingredient (1) and an ingredient (4) were slowly added with stirring and dissolved. After recognizing the dissolution, heating was discontinued, and ingredients (5) to (7) were added to the mixture with stirring at the weight ratios specified in Table 2. The mixture was stirred until it became a uniform liquid. Ingredients (8) to (11) were added

to the mixture at 30 to 40°C and stirred to homogeneity to prepare a shampoo sample. The obtained shampoo sample is named a test prescription S6 of the present invention.

4-d

Shampoo having composition sown in column (D) of Table 2 was prepared by using the cation-modified galactomannan polysaccharide prepared in Example 1 and named test sample 1 and further containing both tcation-modified hydroxyethyl cellulose and amphoteric water-soluble polymer. Ingredient (12) in column (D) of Table 2 was heated to 60°C to which an ingredient (1), ingredient (3) and ingredient (4)) were slowly added with stirring and dissolved. After recognizing the dissolution, heating was discontinued, and ingredients (5) to (7) were added to the mixture with stirring. The mixture was stirred until it became a uniform liquid. Ingredients (8) to (11) were added to the mixture at 30 to 40°C, and the mixture was stirred to homogeneity to prepare a shampoo sample. The obtained shampoo sample is named a test prescription S7 of the present invention.

4-e (Preparation of comparative prescriptions)

To compare the effects of test shampoo preparations containing a cation-modified galactomannan polysaccharide of the present invention, comparative shampoo samples were prepared by using the cation-modified galactomannan polysaccharides prepared in Supplement 1, i.e., the named samples 6 and 7 listed in Table 1. The composition of the comparative shampoo samples was as shown in column (E) of Table 2. Specifically, an ingredient (12) of the comparative shampoo sample in Table 2 was heated to 60°C to which comparative ingredient (2) was slowly added with stirring. The stirring was continued until the substance added was dissolved. After recognizing the dissolution, heating was discontinued, and the ingredients (5) to (7) were added to the mixture with stirring. The mixture was stirred until it became a uniform liquid. Ingredients (8), (10) and (11) were added to the mixture at 30 to 40°C, and the mixture was stirred to homogeneity. The shampoo samples containing sample 6 or 7 in Table 1 were named comparative prescriptions C1 and C2.

TABLE 2. Wash-away product (Shampoo) Prescriptions

| Ingredients \ Weight ratio (%, in terms of solids) | | (A) | (B) | (C) | (D) | Comparative sample (E) | Blank (F) |
|---|---|---|---|---|---|---|---|
| (1) | Test samples (test sample 1 to 4) | 0.7 | | | | 0 | 0 |
| (2) | Comparative samples (comparative sample 6, 7, fenugreek gum) | 0 | | | | 0.7 | 0.0 |
| (3) | Cationic water-soluble polymer (Catinal HC-100) | 0 | 0.4 | 0 | 0.2 | 0 | 0 |
| (4) | Amphoteric water-soluble polymer (Yukaformer SM) | 0 | 0 | 0.4 | 0.2 | 0 | 0 |
| (5) | Polyoxyethylene (3)lauryl ether sulfuric acid sodium | 9.0 | | | | 9.0 | 9.0 |
| (6) | Coconut oil fatty acid amide propylbetaine | 4.5 | | | | 4.5 | 4.5 |
| (7) | Coconut oil fatty acid monoethanol amide | 2.5 | | | | 2.5 | 2.5 |
| (8) | Sodium edetate | 0.1 | | | | 0.1 | 0.1 |
| (10) | Sodium benzoate | 0.1 | | | | 0.1 | 0.1 |
| (11) | Aqueous solution of citric acid (for pH adjustment, pH 5.5-6.0) | As appropriate | | | | As appropriate | As appro-priate |
| (12) | Distilled water | The rest | | | | The rest | The rest |

4-f (Preparation of comparative prescription)

To compare the effects of test shampoo preparations containing a cation-modified galactomannan polysaccharide used in the present invention, a comparative shampoo sample having composition shown in column (E) of Table 2 was prepared by using, instead of the sample 6 used in the above-4-e, an unmodified or intact galactomannan polysaccharide (fenugreek gum (Fenugreek Albumen Powder A manufactured by Air Green Co. Ltd.)) which is the same in preparation proportion as the sample 6. The shampoo sample thus prepared was named comparative prescription C3.

(Evaluation)

In this test, the present test prescriptions S1 to S7 and comparative prescriptions C1, C2 and C3 prepared as described in 4-a to 4-f, and a blank prescription with a composition as shown in column (F) of Table 2 were used. Strands of hairs (total length being 180 mm) weighing 15 g were washed with 1.0 g of each prescription. Later, the strands of hairs were rinsed with running water. The hair strands were left for 24 hours in an atmosphere kept at constant temperature and constant humidity (20°C and 40% RH) to be naturally dried. Then, strength of hair richness was tested with a pure-flexural test equipment (KES-FB2-S, KatoTech). The blank prescription was prepared in such a manner that ingredient (12) in Blank (F) in Table 2 was heated to 60°C to which the ingredients (5) to (7) were added. The mixture was stirred until it became a uniform liquid and cooled. Ingredients (8) to (11) were added to the mixture at 30 to 40°C and the mixture was stirred to homogeneity. The results are shown in Table 3.

With respect to sampoos of the present test prescriptions S1 to S7 and the comparative prescriptions C1 to C3 prepared as described in 4-a to 4-f and the shampoo comprising the composition as shown in Blank (F) of Table 2 the blank prescription, ten testers washed their hair using each of the prescriptions and subjectively assessed softness of hair strand after their hair was dried with a dryer. Evaluation was carried out by the following standard according to the number of the tester who felt a positive softness of hair quality. The results are shown in Table 3.

◎ -- The number of positive testers is eight or more
○ -- The number of positive testers is six to seven
Δ -- The number of positive testers is four to five
× -- The number of positive testers is three or less

TABLE 3. Pure Flexural Test (Flexibility)

| Cationic polymers used | Blank | Test prescriptions | | | | | | | Comparative prescriptions | | |
|---|---|---|---|---|---|---|---|---|---|---|---|
| | | S1 | S2 | S3 | S4 | S5 | S6 | S7 | C1 | C2 | C3 |
| | | | | | | Sample 1 | | | | | |
| | | Sample 1 | Sample 3 | Sample 4 | Sample 5 | Catinal HC-100 | Yuka-former SM | Catinal HC-100 + Yukaformer SM | Sample 6 | Sample 7 | Intact galactomannan polysaccharide (fenugreek gum) |
| Flexural rigidity B-value (gf·cm2/cm) | 0.56 | 0.60 | 0.61 | 0.58 | 0.57 | 0.65 | 0.70 | 0.70 | 0.59 | 1.05 | 0.57 |
| Hysteresis width 2HB-value (gf·cm/cm) | 0.10 | 0.36 | 0.35 | 0.33 | 0.33 | 0.35 | 0.37 | 0.37 | 0.11 | 0.31 | 0.09 |
| Flexibility | × | ◎ | ◎ | ◎ | ◎ | ◎ | ◎ | ◎ | Δ | × | × |

The results of Table 3 indicate that when the test prescriptions S1 to S4 of the present invention containing the cation-modified galactomannan polysaccharide of the samples 1, 3, 4 and 5 in which galactomannan polysaccharide was cation-modified were compared with the blank preparation, there was no large change in flexural rigidity (B-value), but hysteresis width (2HB) was increased in the Sampoos S1 to S4. The comparative prescription C2 containing a cation-modified galactomannan polysaccharide of the sample 7 whose cationic charge density was higher than that of the corresponding cation-modified galactomannan polysaccharide of the present invention showed similar results. However, the flexural rigidity of the comparative prescription C2 was so large that the hairs treated with it became rigidity. On the other hand, with regard to the comparative prescription C1 containing a cation-modified galactomannan polysaccharide of the sample 6 whose cationic charge density was lower than that of the corresponding cation-modified galactomannan polysaccharide of the present invention, and comparative prescription C3 containing an unmodified or intact galactomannan polysaccharide were in the same result. This is probably because when unmodified or intact galactomannan polysaccharide and cation-modified galactomannan polysaccharide whose cationic charge density was lower than the scope of the present invention were used in a wash-away products, absorbability based on inonic property to hair could not be obtained and adsoption of the galactomannan polysaccharide to hair was less, and therefore, their effects were not obtained. In contrast, since the test shampoo prescriptions of the present invention in which galactomannan polysaccharide was cation-modified had adsorbability based on ionic property to hair, flexibility in a wash-away products such as a shmapoo was obtained. As a result, such effects that hysteresis width was improved and that soft feel to hair was given to hair in feel aspect by hand were obtained. Therefore, it was found out that the cation-modified galactomannan polysaccharide of the present invention fully exhibits its effects even when it is added to wash-away product such as shampoos and hair rinses.

It was confirmed that, when a cation-modified galactomannan polysaccharide of the present invention was used in combination with a water-soluble polymer having a conditioning effect, that is, a cation-modified hydroxyethyl cellulose containing nitrogen at 1.8 wt% to which an average of 1.8 mol of ethylene oxide was introduced (Catinal HC-100, Toho Chemical Industry) or a copolymer of N-methacryloyloxyethyl N,N-dimethyl ammonium-$\alpha$-methylcarboxylbetaine and alkyl methacrylate (Yukaformer SM, Mitsubishi Chemicals Co.), there was no difference in hysteresis width

(2HB), and even used in combination, noly only the effects of the present invention is not lost, but also flexural rigidity was appropriately improved, whereby providing richness of hair.

Example 5. Flexibility to hair (Part 2)

(Evaluation)

The hair flexibility enhancing effect on the damaged hairs by the cation-modified galactomannan polysaccharide of the present invention was tested using wash-away products containing a cation-modified galactomannan polysaccharide of the present invention, that is, the test prescriptions S1 to S7 and comparative prescriptions C1 to C3 prepared in Example 4. The same strands of hairs as the one used in Example 4 were immersed in a bleaching solution which comprised a 6% $H_2O_2$ and 3% aqueous ammonium at 2:1 (w/w): the hair strand was immersed for 60 minutes in the bleaching solution whose weight against that of the hair strand was adjusted to 100:1 and whose temperature was kept at 40°C. The hair strand was rinsed with warm water, and dried with a dryer. The strands of hairs which had been seriously damaged by this bleaching treatment were used for the test. The strands of damaged hairs (total length being 180 mm) weighing 15 g were washed with 1.0 g of each prescription of the test prescriptions S1 to S7 prepared as described in 4-a to 4-d, comparative prescriptions C1 to C3 prepared as described in 4-e to 4-f, and a shampoo comprising the composition under Blank (F) in Table 2 as a blank prescription were used.

Later, the strands of hairs were rinsed with running water. The hair strands were left for 24 hours in an atmosphere kept at constant temperature and constant humidity (20°C and 40% RH) to be naturally dried. Then, the strength of hair richness was tested with a pure-flexural test equipment (KES-FB2-S, KatoTech). The results are shown in Table 3. The blank prescription was obtained in such a manner that ingredient (12) under column (F) of Table 2 was heated to 60°C to which the ingredients (5) to (7) were added, the mixture was stirred until it became a uniform liquid and after the mixture was cooled, ingredients (8) to (11) were added at 30 to 40°C, and the mixture was stirred to homogeneity.

TABLE 4. Evaluation of Pure-Flexural Test of Damaged Hairs (Flexibility)

| | | Test prescriptions | | | | | | | Comparative prescriptions | | |
|---|---|---|---|---|---|---|---|---|---|---|---|
| | | S1 | S2 | S3 | S4 | S5 | S6 | S7 | C1 | C2 | C3 |
| | | | | | | Sample 1 | | | | | |
| Cationic polymers used | Blank | Sample 1 | Sample 3 | Sample 4 | Sample 5 | Catinal HC-100 | Yuka-former SM | Catinal HC-100 + Yukaformer SM | Sample 6 | Sample 7 | Intact galactomannan polysaccharide (fenugreek gum) |
| Flexural rigidity B-value (gf·cm2/cm) | 0.32 | 0.59 | 0.62 | 0.60 | 0.63 | 0.68 | 0.74 | 0.69 | 0.40 | 1.16 | 0.33 |
| Hysteresis width 2HB-value (gf·cm/cm) | 0.05 | 0.34 | 0.31 | 0.35 | 0.36 | 0.36 | 0.38 | 0.38 | 0.13 | 0.22 | 0.05 |

In a case that evaluation was carried out on damaged hair from the results of Table 4, when the test prescriptions S1 to S4 and the Blank were compared, both flexural rigidity (B-value) and hysteresis width (2HB) were improved. The comparative prescription C2 containing a cation-modified galactomannan polysaccharide of the Sample 7 with cationic charge density higher than the scope of the present invention showed similar result, but the flexural rigidity of the prescription C2 was so large that the hairs treated with it suggested rigidity. On the other hand, the comparative prescription C1 containing a cation-modified galactomannan polysaccharide of the Sample 6 whose cationic charge density is lower than the scope of the present invention improved slightly its effects on the flexural rigidity and hysteresis width, but they were not sufficiently large to be notable. With regard to the comparative prescription C3 containing an unmodified

or intact galactomannan polysaccharide, the measurement results were found to be practically the same as those of the blank preparation. Both the difference between the flexural rigidity averaged for the present test prescriptions and the flexural rigidity of the blank preparation, and the difference between the hysteresis width averaged for the present test prescriptions and the hysteresis width of the blank prescription were larger when the prescriptions were tested on the damaged hairs than on the normal hairs untreated with bleach described in Example 4. This suggests that the test prescriptions containing the cation-modified galactomannan polysaccharide of the present invention are also effective in treating damaged hairs.

It was confirmed that, when a cation-modified galactomannan polysaccharide of the present invention was used in combination with a water-soluble polymer having a conditioning effect, that is, a cation-modified hydroxyethyl cellulose containing nitrogen at 1.8 wt% to which an average of 1.8 mol of ethylene oxide was introduced (Catinal HC-100, Toho Chemical Industry) or a copolymer of N-methacryloyloxyethyl N,N-dimethyl ammonium-α-methylcarboxylbetaine and alkyl methacrylate (Yukaformer SM, Mitsubishi Chemicals Co.), there was no difference in hysteresis width, and even used in combination, noly only the effects of the present invention is not lost, but also flexural rigidity was appropriately improved, whereby providing richness of hair.

Example 6: Emulsifiability

The emulsifiability of the cation-modified galactomannan polysaccharides (sample Nos. 1, 2, 4 and 5 in Talbe 1) of the present invention were determined by the following method. In room temperature (25°C), 10 ml of n-butanol as an oil is added to 40 ml of an experiment solution (0.125% sample aqueous solution) in a stopcock-provided messcylinder having an internal volume of 100 ml. The test solution was shaken and agitated for 30 seconds and n-butanol was emulsified. Thereafter, volumes of n-butanol separated after 30 minutes and 3 hours were measured. The result is shown in Table 5. The numerical values in Table 5 shows that the smaller the numerical values are, the larger the emulsifiability is. For the comparison, similar emulsifiability experiments were conducted to cation-modified guar gum (Samples No. 8 and No. 9 in Table 1) obtained by cation-modifying guar gum which is the same galactomannan polysaccharide and has different composition ratio of mannose and galactose (the composition ratio of mannose and galactose is 2:1), cation-modified locust-bean gum obtained by cation-modifying locust-bean gum which is the similar galactomannan polysaccharide and has different composition ratio of mannose and galactose (the composition ratio of mannose and galactose is 4:1), cation-modified hydroxyethyl cellulose (Cational HC-100 manufactured by Toho Chemical Industry, Ltd.), as other cationic polymer, having 1.8 mol of average addition number of ethylenoxide and 1.8 wt% of nitrogen content, and unmodified or intact galactomannan polysaccharide (fenugreek gum, "Fenugreek Albumen Powder A" manufactured by Air Green Co., Ltd.). The result is shown in Table 5.

TABLE 5. Test Results of Emulsifiability

| Test cationic polymers | | Blank | Test prescriptions | | | | Comparative prescriptions | | | |
|---|---|---|---|---|---|---|---|---|---|---|
| | | | Sample 1 | Sample 2 | Sample 4 | Sample 5 | Sample 8 | Sample 9 | Cational HC-100 | intact galactomannan polysaccharide (fenugreek gum) |
| Butanol layer (transparent layer) ml | After 30 minutes | 10 | 0 | 0 | 0 | 0 | 0.5 | 0.5 | 0.5 | 0.5 |
| | After 3 hours | 10 | 0.5 | 0 | 0 | 0 | 2.5 | 2.5 | 3 | 1.5 |

From the result of Table 5, it was proved that the cation-modified galactomannan polysaccharides of the present invention increased emulsifiability in comparison with fenugreek gum which is unmodified or intact galactomannan polysaccharide. This is a result that due to cation-modification, alkyl group in the cation-modifying group was acted as hydrophobic group. Even though the guar gaum and the locust-bean gum which are the same galactose polysaccharide, the cation-modified guar gum obtained by cation-modifying the guar gum having poor emulsifiability and the composition ratio (2: 1) of mannose as the main chain and galactose as the side chain, and the the cation-modified locust-bean gum obtained by cation-modifying locust-bean gum having poor emulsifiability and the constituion ratio (4:1) of mannose as the main chain and the galactose as the side chain were proved to have poor emulsifiability. As a consequence of this, when the cation-modified galactosemannan polysaccharide of the present invention is used to a hair care composition and a skin cosmetic composition, feeling improvement accompanied by emulsifiability is expected.

Hair shampoo

(Preparation)

Example 6

6-a

The cation-modified galactomannan polysaccharides prepared in Examples 1, 2 and 3 and named Samples 1-5 were used to prepare shampoo samples with compositions as shown in column (A) of Table 6. The shampoo sample was prepared as follows. Ingredient (14) in column (A) of Table 6 was heated to 65°C to which ingredient (1) was slowly added with stirring, and mixture was stirred until it was dissolved. After recognizing the dissolution, heating was discontinued, and ingredients (5) to (10) were added to the mixture with stirring. The mixture was stirred until it became a uniform liquid, to which ingredients (11) to (13) were added, and the mixture was stirred to homogeneity. Thus, five kinds of shampoo samples with compositions as shown in column (A) of Table 6 were obtained. The five shampoo samples containing the cation-modified galactomannan polysaccharides represented by samples 1-5 correspond to the named test prescriptions 1-5 in Table 1, respectively.

6-b

The cation-modified galactomannan polysaccharide prepared in Example 1 and named Sample 1 and a cation-modified hydroxyethyl cellulose containing nitrogen at 1.8 wt% to which an average of 1.8 mol of ethylene oxide was introduced (Catinal HC-100, Toho Chemical Industry) represented as a cation-modified water-soluble polymer A in Table 6 were combined at the weight ratios specified in column (B) of Table 6, and used as a test sample. Ingredient (14) in column (B) of Table 6 was heated to 65°C to which ingredient (1) and ingredient (3) were slowly added with stirring. The stirring was continued until the substances added were dissolved. After recognizing the dissolution, heating was discontinued, and the ingredients (5) to (10) were added to the mixture with stirring. The mixture was stirred until it became a uniform liquid, to which ingredients (11) to (13) were further added at 30 to 40°C, and the mixture was stirred to homogeneity. The obtained shampoo sample was named test prescription 6.

6-c

The same procedures as described above were employed except that a copolymer of diallyldimethyl ammonium chloride and acryl amide (Merquat 550, Nalco) was used instead of the cation-modified water-soluble polymer A, to provide a shampoo sample with a composition as shown in column (C) of Table 6. This was named test prescription 7.

6-d

The cation-modified galactomannan polysaccharide prepared in Example 1 and named Sample 1 and a copolymer of N-methacryloyloxyethyl N,N-dimethyl ammonium-$\alpha$-methylcarboxylbetaine and alkyl methacrylate (Yukaformer SM, Mitsubishi Chemicals Co.) as an amphoteric water-soluble polymer were combined at the weight ratio specified in column (D) of Table 6, and used as a test sample. Ingredient (14) in column (D) of Table 6 was heated to 65°C to which ingredient (1) and ingredient (4) were slowly added with stirring. The stirring was continued until the substances added were completely dissolved. Then, heating was discontinued, and the ingredients (5) to (10) were added to the mixture with stirring. The mixture was stirred until it became a uniform liquid. Ingredients (11) to (13) were added to the mixture at 30 to 40°C, and the mixture was stirred to homogeneity. The obtained shampoo sample was named test prescription 8.

6-e

The cation-modified galactomannan polysaccharide prepared in Example 1 and named Sample 1, the cationic water-soluble polymer and the amphoteric water-soluble polymer were combined at the weight ratios specified under (E) in Table 6, and used as a test sample. Ingredient (14) in column (E) of Table 6 was heated to 65°C to which ingredient (1), ingredient (3) and ingredient (4) were slowly added with stirring. The stirring was continued until the substances added were cdissolved. After recognizing the dissolution, heating was discontinued, and the ingredients (5) to (10) were added to the mixture with stirring. The mixture was stirred until it became a uniform liquid. Ingredients (11) to (13) were added to the mixture at 30 to 40°C,, and the mixture was stirred to homogeneity. The obtained shampoo sample was named test prescription 9.

6-f (Preparation of comparative prescriptions)

To compare the effects of the test shampoo preparations containing the cation-modified galactomannan polysaccharide of the present invention, comparative shampoo samples were prepared by using the cation-modified galactomannan polysaccharides prepared in Supplement 1, i.e., the named Samples 6 and 7 listed in Table 1. The composition of the comparative shampoo samples was as shown in column (G) of Table 6. Ingredient (14) in column (G) of Table 6 was heated to 65°C to which ingredient (2) was slowly added with stirring. The stirring was continued until the substance added was dissolved. After recognizing the dissolution, heating was discontinued, and the ingredients (5) to (10) were added to the mixture with stirring. The mixture was stirred until it became a uniform liquid. Ingredients (11) to (13) were added to the mixture at 30 to 40°C, and the mixture was stirred to homogeneity. The shampoo samples thus obtained were named comparative prescriptions 1 and 2.

TABLE 6. Prescription of Shampoo Preparations

| Ingredients \ Weight ratio (%, in terms of solids) | | (A) | (B) | (C) | (D) | (E) | Reference prescription (F) | Comparative prescriptions (G) |
|---|---|---|---|---|---|---|---|---|
| (1) | Test samples (samples 1-5) | 0.6 | | | | | 0 | 0 |
| (2) | Comparative samples (sample 6-9, Catinal HC-100) | 0 | | | | | 0 | 0.6 |
| (3) | Cationic water-soluble polymer A (Catinal HC-100) | 0 | 0.4 | 0 | 0 | 0.2 | 0 | 0 |
| | Cationic water-soluble polymer B (Merquat 550) | 0 | 0 | 0.4 | 0 | 0 | 0 | 0 |
| (4) | Amphoteric water-soluble polymer (Yukaformer SM) | 0 | 0 | 0 | 0.4 | 0.2 | 0 | 0 |
| (5) | Polyoxylethylene(1,5)laurylether sulfuric acid triethanol amine | 6.0 | | | | | 6.0 | 6.0 |
| (6) | Polyoxyethylene(3)laurylether sulfuric acid sodium | 3.0 | | | | | 3 | 3.0 |
| (7) | Coconut oil fatty acid amide propylbetaine | 4.5 | | | | | 4.5 | 4.5 |
| (8) | Coconut oil fatty acid monoethanol amide | 2.5 | | | | | 2.5 | 2.5 |
| (9) | Dipropylene glycol | 3.0 | | | | | 3.0 | 3.0 |
| (10) | Ethylene glycol distearate | 1.0 | | | | | 1.0 | 1.0 |
| (11) | Sodium edetate | 0.1 | | | | | 0.1 | 0.1 |
| (12) | Sodium benzoate | 0.1 | | | | | 0.1 | 0.1 |
| (13) | Aqueous solution of citric acid (for pH adjustment, pH 5.5-6.0) | As appropriate | | | | | As appropriate | As appropriate |
| (14) | Distilled water | The rest | | | | | The rest | The rest |

6-g (Preparation of comparative prescription)

To further compare effects, shampoos having the composition shown in comparative prescription (G) in Table 6 were prepared in similairty with the above section 6-f by using, instead of cation-modified galactomannan polysaccharide in the above section 6-f, cation-modified galactomannan polysaccharide in which the composition ratio of mannose as a main chain and galacose as a side chain is different from that of the present invention, i.e., Samples 8 and 9 in Table 1 which were obtained in Comparative Example 1. The obtained products were named as comparative prescriptions 3 and 4.

6-h (Preparation of comparative prescription)

To further compare the effects with other cationic, a comparative shampoo sample was prepared by using, instead of the cation-modified galactomannan polysaccharide in section 6-f, a cation-modified hydroxyethyl cellulose containing nitrogen at 1.8 wt% to which an average of 1.8 mol of ethylene oxide was introduced (Catinal HC-100, Toho Chemical Industry). The ingredients shown in column (G) of Table 6 were treated in the same manner as described in section 6-f to provide the comparative shampoo preparation which was named comparative prescription 5.

(Evaluation)

Example 7

In this test, test prescriptions 1 to 9 prepared as described in 6-a to 6-e, and a reference prescription were used. The reference prescription with a composition as shown in column (F) of Table 6 was obtained by using ingredients similar to those used for the production of test and comparative prescriptions and treating them similarly, except that they did not contain any cationic polymer.

Of test prescriptions 1 to 9, and the reference prescription (as a reference), ten testers washed their hair using each of the shampoo prescriptions, and dried their hair with a dryer. The testers compared the conditioning effects of the respective test prescriptions as described below as to use feeling during use and after dried, with those of the reference prescription. The conditioning effects compared consisted of:

* lathering at washing of hair
* existence of conditioning effects during use (easy finger passing and feeling of hair)
* existence of conditioning effects after use (dried hair) (comb-passing feel, feeling of hair), and hair flexibility.

The tester's evaluation was scored according to the following five rank scale. For each of the test prescriptions, the scores of the ten testers were summed as in shown in Tables 7 and 8, and the sum of ten testers' evaluation was taken as its overall evaluation result. The evaluation results of the test prescriptions are shown in Table 9. The reference prescription (F) was prepared in such a manner that ingredient (14) in reference prescription (F) of Table 6 was heated to 65°C to which the ingredients (5) to (10) were added. The mixture was stirred until it became a uniform liquid. To the mixture which was cooled to 30 to 40°C, ingredients (11) to (13) were added, and the mixture was stirred to homogeneity.

TABLE 7. Ranking Scale for Evaluating Test Prescriptions (During Use)

| | Test properties | | |
|---|---|---|---|
| Score | Lathering | Easy finger passing at washing of hair | Hand texture of wet hair |
| +2 | Higher | Better | Smoother |
| +1 | Slightly higher | Slightly better | Slightly smoother |
| 0 | The same | The same | The same |
| -1 | Slightly lower | Slightly worse | Slightly more sticky |
| -2 | Lower | Worse | More sticky |

TABLE 8. Ranking Scale for Evaluating Test Prescriptions (After Use)

| | Test properties | | |
|---|---|---|---|
| Score | Moist feeling | Easy comb-passing | Hair flexibility |
| +2 | Better | Better | Soft |
| +1 | Slightly better | Slightly better | Slightly soft |
| 0 | The same | The same | The same |
| -1 | Slightly dry feeling | Slightly worse | Slight rigidity |
| -2 | Dry feeling | Worse | Rigidity |

Comparative Example 2

With regard to comparative prescriptions 1 to 5 prepared as described in 6-f to 6-h, their conditioning effects were compared with those of the reference prescription in the same manner as in Example 7. The thus obtained evaluation results of comparative prescriptions 1 to 5 are shown in Table 9.

TABLE 9. Evaluation Results of Shampoo Preparations

| | | Test prescriptions | | | | | | | | | Comparative prescriptions | | | | |
|---|---|---|---|---|---|---|---|---|---|---|---|---|---|---|---|
| | | 1 | 2 | 3 | 4 | 5 | 6 | 7 | 8 | 9 | 1 | 2 | 3 | 4 | 5 |
| Test cationic polymers | | Sample 1 | Sample 2 | Sample 3 | Sample 4 | Sample 5 | Sample 1<br>Catinal HC-100 | Sample 1<br>Merquat 550 | Sample 1<br>Yuka-former SM | Sample 1<br>Catinal HC-100<br>Yuka-former SM | Sample 6 | Sample 7 | Sample 8 | Sample 8 | Catinal HC-100 |
| During use | Lathering | 14 | 13 | 13 | 13 | 13 | 14 | 14 | 13 | 15 | 5 | -2 | 12 | 8 | 11 |
| | Easy finger passing at washing hair | 13 | 14 | 13 | 14 | 12 | 15 | 14 | 14 | 15 | -10 | -7 | 7 | 13 | 12 |
| | Texture of wet hairs | 12 | 13 | 12 | 14 | 14 | 15 | 14 | 14 | 14 | -8 | -6 | 7 | 10 | 12 |
| After use | Moist feeling | 14 | 13 | 13 | 14 | 13 | 14 | 14 | 15 | 15 | 2 | 0 | 7 | 8 | 5 |
| | Easy comb-passing | 13 | 13 | 12 | 14 | 12 | 14 | 15 | 14 | 14 | 1 | -6 | 11 | 8 | 6 |
| | Hair flexibility | 13 | 13 | 12 | 12 | 12 | 13 | 13 | 13 | 13 | 1 | 5 | 5 | 4 | 2 |

The results of Table 9 indicate that the inventive cation-modified galactomannan polysaccharide whose cationic charge density is in the range of 0.1 to 3.0 meq/g, when added to a shampoo preparation, are superior in conditining effects such as lathering at washing hair, easy-finger passing and hand texture during use, and conditioning effects of moist feeling and easy comb-passing after use.

On the other hand, the comparative prescriptions 3 and 4 containing samples 8 and 9, respectively, which were the same galactomannan polysaccharide and were prepared by cation-modifying guar gum and locus gum in which the composition ratio of mannose and galactose were different showed some conditioning effects such as "easy finger passing at washing hair". However, they were poor in moist feeling and flexibility. When the test prescriptions are compared with comparative prescription 5 which contains a conventional conditioning agent, that is, cation-modified hydroxyethyl cellulose (Catinal HC-100), the former have conditioning effects better than or equal to those of the test prescriptions during use, and after use, confer moist feeling and flexibility which the cation-modified galactomannan polysaccharide of the present invention has, in comprison with the conventional conditioning agents have not such moist feeling and flexibility. By using a cationic water-soluble polymer (Catinal HC-100, Merquat 550) and/or an amphoretic water-soluble polymer (Yukaformer SM) together with the compounding ingredients, the conditioning effects of the test prescriptions were found to be enhanced without disturbing the performance possesed by the cation-modified galactomannan polysaccharide of the present invention.

(Preparation Examples 1-3)

Preferred shampoo compositions according to the present invention will be described below.

TABLE 10. Preparation Example 1. Shampoo (1)

| Ingredients | Weight ratio (%) |
|---|---|
| Coconut oil fatty acid methyl taurine sodium | 9.0 |
| Coconut oil fatty acid amide propylbetaine | 3.0 |
| Lauric acid propylene glycol | 0.9 |
| Coconut oil fatty acid diethanol amide | 1.0 |
| Polyoxyethylene(4)alkyl(C12-14)sulfosuccinic acid disodium | 2.0 |
| Cationic cellulose | 0.5 |
| Test prescription (sample 2) | 0.2 |
| Propylene glycol | 0.3 |
| Sodium chloride | 1.5 |
| Perfume, pigment, preservative | As appropriate |
| Metal ion sealant, pH adjuster | As appropriate |
| Purified water | The rest |

Purified water was heated to 70°C, to which other ingredients were added and dissolved to homogeneity. The mixture was then cooled.

TABLE 11. Preparation Example 2. Shampoo (2)

| Ingredients | Weight ratio (%) |
|---|---|
| Distearic acid ethylene glycol | 2.0 |
| Coconut oil fatty acid methyl taurine sodium | 7.0 |
| Coconut oil fatty acid sarcosine sodium | 1.0 |
| Perm kernel fatty acid amide propylbetaine | 5.0 |
| Test prescription (sample 1) | 0.5 |
| Coconut oil fatty acid diethanol amide | 3.0 |
| Stearic acid dimethylaminopropyl amide | 0.3 |
| Citric acid | 0.55 |
| Sodium chloride | 1.2 |
| Phenoxyethanol | 0.1 |
| Sodium benzoate | 0.3 |
| Disodium 2 edetate | 0.05 |
| Purified water | The rest |
| Perfume | 0.3 |

Purified water was heated to 70°C, to which other ingredients were added and dissolved to homogeneity. The mixture was then cooled.

TABLE 12. Preparation Example 3. Shampoo (3)

| Ingredients | Weight ratio (%) |
|---|---|
| Polyoxyethylene(2)laurylether sulfuric acid sodium | 10.0 |
| Polyoxyethylene coconut oil fatty acid monoethanolamide sulfuric acid sodium | 2.0 |

Table continued

| Ingredients | Weight ratio (%) |
|---|---|
| Distearic acid ethylene glycol | 1.8 |
| Lauric acid propylene glycol, | 2.0 |
| Coconut oil fatty acid amide propylbetaine | 2.5 |
| Laurylhydroxysulfobetaine | 1.5 |
| Methylpolysiloxane micro-emulsion | 1.0 |
| Test prescription (sample 4) | 0.3 |
| Polyquaternium-10 | 0.1 |
| Propylene glycol | 2.0 |
| Coconut oil fatty monoethanolamide | 0.4 |
| Glyceryl oleate | 0.2 |
| Citric acid | 0.8 |
| Alginine | 0.1 |
| Sodium chloride | 0.4 |
| Phenoxyethanol | 0.1 |
| Sodium benzoate | 0.3 |
| Disodium 2 edetate | 0.05 |
| Purified water | The rest |
| Perfume | 0.3 |

Purified water was heated to 70°C, to which other ingredients were added and dissolved to homogeneity. The mixture was then cooled.

Hair Rinse

(Preparation)

Example 8

8-a

The cation-modified galactomannan polysaccharides prepared in Example 1 and named Sample 1 was used to prepare hair rinse samples whose composition is as shown in column (A) of Table 13 below. The hair rinse sample contained an amideamine compound, a behenic acid dimethylaminopropylamide citric acid salt which had been neutralized by citric acid as a neutralizer, and a higher alcohol (cetanol). The hair rinse sample was prepared as follows. Ingredients (5) to (10) specified in column (A) of Table 13 were heated to 80°C and stirred until the mixture became a uniform liquid. Ingredient (1) was previously added with stirring to ingredient (12), and the mixture was stirred until it was dissolved. The solution, after being heated to 80°C, was added to the above liquid with stirring, and ingredient (11) was added to the mixture while the mixture was being cooled, and the mixture was stirred until it became a uniform solution. Thus, hair rinse sample with compositions as shown in column (A) of Table 13 were obtained. The hair rinse samples containing the cation-modified galactomannan polysaccharides represented by Sample 1 in Table 1 was named test prescription 10.

8-b

The cation-modified galactomannan polysaccharides prepared in Examples 1 and 2 and named Samples 2 to 4 were used to prepare hair rinse samples whose composition is as shown in column (B) of Table 13 below. The hiar rinse sample contained an organic acid salt (behenic acid dimethylaminoporplyamide and citric acid salt) of an amideamine compound and a higher alcohol (cetanol). The hair rinse sample was prepared as follows. Ingredients (5) to (10) specified in column (B) of Table 13 were heated to 80°C and stirred until the mixture became a uniform liquid. Ingredient (1) was previously added with stirring to ingredient (12), and the mixture was stirred until it was dissolved. The solution, after being heated to 80°C, was added to the above liquid with stirring, and ingredient (11) was added to the mixture while the mixture was being cooled, and the mixture was stirred until it became a uniform solution. Thus, hair rinse samples with compositions as shown in column (B) of Table 13 were obtained. The hair rinse samples containing the cation-modified galactomannan polysaccharides represented by samples 2 to 4 in Table 1 was named test prescriptions 11 to 13, respectively.

8-c

The cation-modified galactomannan polysaccharides prepared in Example 1 and named Sample 1 and a cation-modified hydroxyethyl cellulose containing nitrogen at 1.8 wt% into which an average of 1.8 mol of ethylene oxide was introduced (Catinal HC-100, Toho Chemical Industry) were used at the weight ratios specified column (C) of Table 13 to give a mixture to which ingredients shown in column (C) of Table 13 were added, and the resulting product was used as a test hair rinse sample. Ingredients (5) to (10) specified in column (C) of Table 13 were heated to 80°C and stirred until the mixture became a uniform liquid. Ingredient (1) and ingredient (3) were previously added with stirring to ingredient (12), and the mixture was stirred until it was dissolved. The solution, after being heated to 80°C, was added to the above liquid with stirring, and the mixture was stirred until it became a uniform solution. Then, ingredient (11) was added to the mixture while the mixture was being cooled. The mixture was stirred until it became a uniform solution. The obtained hair rinse sample was named test prescription 14.

8-d

The cation-modified galactomannan polysaccharides prepared in Example 1 and named Sample 1 and an amphoteric water-soluble polymer or a copolymer of N-methacryloyloxyethyl N,N-dimethyl ammonium-$\alpha$-methylcarboxylbetaine and alkyl methacrylate (Yukaformer SM, Mitsubishi Chemicals Co.) were used to give a mixture to which ingredients shown in column (D) of Table 13 were added and the resulting product was used as a test hair rinse sample. Ingredients (5) to (10) specified in column (D) of Table 13 were heated to 80°C and stirred until the mixture became a uniform liquid. Ingredient (1) and ingredient (4) were previously added with stirring to ingredient (12), and the mixture was stirred until it was dissolved. The solution, after being heated to 80°C, was poured into the above liquid with stirring, and the mixture was stirred until it became a uniform solution. Then, ingredient (11) was added to the mixture while the mixture was being cooled. The mixture was stirred until it became a uniform solution. The obtained hair rinse sample was named test prescription 15.

8-e (Preparation of comparative prescriptions)

To compare the effects of the test hair rinse preparations containing the cation-modified galactomannan polysaccharide of the present invention, comparative shampoo samples were prepared by using the cation-modified galactomannan polysaccharides prepared in Supplement 1, i.e., the named Samples 6 and 7 listed in Table 1. The composition of the comparative hair rinse samples was as shown in column (F) of Table 13. Specifically, ingredients (5) to (10) specified in column (F) of Table 13 were heated to 80°C and stirred until the mixture became a uniform liquid. Ingredient (2) was previously added with stirring to ingredient (12), and the mixture was stirred until it was completely dissolved. The solution, after being heated to 80°C, was added to the above liquid with stirring, and the mixture was stirred until it became a uniform solution. Then, ingredient (11) was added to the mixture while the mixture was being cooled. The mixture was stirred until it became a uniform solution. The obtained hair rinse samples containing Sample Noss 6 and 7 in Table 1 were named comparative prescriptions 6 and 7, respectively.

8-f (Preparation of comparative prescription)

To further compare effects, rinses having the composition shown in comparative prescription (F) in Table 13 were prepared in similarity with the above section 8-e by using, instead of the Samples 6 and 7, cation-modified galactomannan polysaccharide in which the composition ratio of mannose as a main chain and galacose as a side chain is different from that of the present invention, i.e., Samples 8 and 9 in Table 1 which were obtained in Comparative Example 1. The obtained rinses were named as comparative prescriptions 8 and 9.

TABLE 13. Prescription of Rinse Preparations

| Ingredients \ Weight ratio (%, in terms of solids) | | (A) | (B) | (C) | (D) | Reference prescription (E) | Comparative prescription (F) |
|---|---|---|---|---|---|---|---|
| (1) | Test samples (sample 1-4) | 1.0 | | | | 0 | 0 |
| (2) | Comparative samples (sample 6-9) | 0 | | | | 0 | 1.0 |
| (3) | Cationic water-soluble polymer (Catinal HC-100) | 0 | 0 | 0.5 | 0 | 0 | 0 |
| (4) | Amphoteric water-soluble polymer (Yukaformer SM) | 0 | 0 | 0 | 0.5 | 0 | 0 |
| (5) | Behenic acid dimethylaminopropylamide citric acid salt | 2.0 | | | | 2.0 | 2.0 |
| (6) | Cetyl lactate | 2.0 | | | | 2.0 | 2.0 |
| (7) | Polyoxyethylene(4)stearylether | 0.6 | 1.0 | | | 1.0 | 1.0 |
| (8) | Isopropyl palmitate | 1.0 | | | | 1.0 | 1.0 |
| (9) | Cetanol | 6.0 | | | | 6.0 | 6.0 |
| (10 | Paraoxybenzoic acid methyl | 0.1 | | | | 0.1 | 0.1 |
| (11) | Aqueous solution of citric acid (for pH adjustment, pH 4.0-4.5) | As appropriate | | | | As appropriate | As appropriate |
| (12) | Distilled water | The rest | | | | The rest | The rest |

(Evaluation)

Example 9

In this test, performance evaluation shown in the following items was conducted with respect to the test prescriptions 10 to 15 prepared as described in 8-a to 8-d (rinse) by using ten testers.

Performance evaluation system used rinse having composition shown in a reference prescription (E) in Table 13 containing no cationic polymer in its ingredient and rinses to be evaluated.

Of test prescriptions 10 to 15, and the reference prescription having no cationic polymer, ten testers used each of the prescriptions, and dried the hair with a dryer. The testers compared the conditioning effects of the respective test prescriptions as described below during and after use with those of the reference prescription (E). The conditioning effects compared consisted of:

* existence of conditioning effects of dried hairs (easy comb-passing and frictional feel)
* hair flexibility

The tester's evaluation was scored according to the following rank scale in Table 14.

For each of the test prescriptions, the scores of the ten testers were summed and the sum of the ten testers' evaluation was taken as its overall evaluation result by the system shown in Table 14. The evaluation results of the test prescriptions are shown in Table 15.

Ingredients (5) to (10) specified in column (E) of Table 13 were heated to 80°C and stirred until the mixture became a uniform liquid. Ingredient (12), after being heated to 80°C, was added to the above liquid with stirring, and the mixture was stirred until it became a uniform solution. Then, ingredient (11) was added to the mixture while the mixture was being cooled. The mixture was stirred until it became a uniform solution.

Comparative Example 3

With regard to comparative prescriptions 6 to 9 prepared as described in 8-e and 8-f, their conditioning effects were compared with those of the reference prescription in the same manner as in Example 9. The thus obtained evaluation results of comparative prescriptions 6 to 9 are shown in Table 15.

TABLE 14. Ranking Scale for Evaluating Test Prescriptions

| | Test properties | |
|---|---|---|
| Score | Comb passing | Frictional feel |
| +2 | Better | Smaller |
| +1 | Slightly better | Slightly smaller |
| 0 | The same | The same |
| -1 | Slightly worse | Slightly larger |
| -2 | Worse | Larger |
| | Test properties | |
| Score | Moist feeling | Hair flexibility |
| +2 | Better | Higher |
| +1 | Slightly better | Slightly higher |
| 0 | The same | The same |
| -1 | Slightly dry feeling | Slightly lower |
| -2 | Dry feeling | Lower |

The results of Table 15 indicate that the hair rinse preparations which contain, in addition to the cation-modified galactomannan polysaccharide of the present invention whose cationic charge density is in the range of 0.1 to 3.0 meq/g, an amideamine compound, neutralizer and higher alcohol (cetanol) not only exhibit excellent hair conditioning effects but also enhance hair flexibility. Furthermore, in the prescription 10 in which polyoxyethylene(4)stearyl-ether (ingredient (7) in Table (3)) which is an emulsifier was reduced, conditioning effects such as easy comb-passing, moist feeling and flexibility which better than or equal to the comparative prescriptions, whereby the emulsifiability of the present invention was determined.

Furthermore, when the test hair rinse preparations containing the cation-modified galactomannan polysaccharide of the present invention, amideamine compound, neutralizer and higher alcohol are compared with Samples 8 and 9, i.e., cation-modified guar gum and cation-modified locust-bean gum prepared by cation-modifying guar gum and locaust-bean gum which are the same galactomannan polysaccharide, but have different composition ratio of guar gum and locust-bean gum, it is found that the former have conditioning effects better than or equal to those of the latter, and confer more improved flexibility after use than the latter because oil moiety and the like remain in hair during rinse-treatment remained appropriately. With regard to test prescriptions 14 and 15 which contain, in addition to the cation-modified galactomannan polysaccharide of the present invention, a cationic water-soluble polymer or an amphoretic water-soluble polymer, the conditioning effects of the test prescriptions were found to be enhanced without disturbing the performance possessed by the cation-modified alginic derivative of the invention.

TABLE 15. Evaluation Results of Rinse Preparations

| Test cationic polymers | | Test prescriptions | | | | | | Comparative prescriptions | | | |
|---|---|---|---|---|---|---|---|---|---|---|---|
| | | 10 | 11 | 12 | 13 | 14 | 15 | 6 | 7 | 8 | 9 |
| | | Sample 1 | Sample 2 | Sample 3 | Sample 4 | Sample 1<br>Catinal HC-100 | Sample 1<br>Merquat 550 | Sample 6 | Sample 7 | Sample 8 | Sample 9 |
| Properties of dried hairs | Comb-passing | 8 | 11 | 10 | 12 | 12 | 13 | 0 | -2 | 8 | 7 |
| | Frictional feel | 7 | 12 | 12 | 13 | 14 | 13 | -4 | -1 | 6 | 7 |
| | Moist feeling | 7 | 11 | 12 | 12 | 13 | 12 | 0 | 8 | 4 | 5 |
| | Flexibility | 7 | 12 | 12 | 11 | 13 | 14 | 1 | 5 | 4 | 5 |

(Preparation Examples 4-6)

Preferred hair treatment compositions such as hair rinses, hair conditioners, etc., according to the present invention which require conditioning effects will be described below.

TABLE 16. Preparation Example 4. Rinse (1)

| Ingredients | Weight ratio (%) |
|---|---|
| Isopropyl palmitate | 3.0 |
| Cetyl lactate | 3.0 |
| Cetyl alcohol | 2.7 |
| Stearyl alcohol | 2.5 |
| Hydroxypropyltrimonium hydrolyzed keratin | 0.3 |
| Stearyltrimethylammonium chloride | 0.7 |
| glycerin | 0.45 |
| Isocetyl myristate | 0.1 |
| Mink fat fatty acid ethyl | 0.2 |
| Self-emulsified monostearic acid glycerin | 0.2 |
| Test prescription (sample 1) | 1.2 |
| Silicone oil | 2.0 |
| Perfume, Pigment, Preservative | As appropriate |
| Purified water | The rest |

To purified water in Table 16 were adde the cation-modified galactomannan polysaccharide of the present invention, stearyltrimethylammonium chloride, glycerin, hydroxypropyltrimonium hydrolyzed keratin and pigment, and the mixture was maintained at 75°C (water phase). The remaining ingredients in Table 16 were mixed and heated so that they were dissolved, and the mixture was maintained at 75°C (oil phase). The water phase was combined with the oil phase, and the mixture was spun with a homogenizer to be emulsified. Then, the emulsion was cooled with stirring.

TABLE 17. Preparation Example 5. Rinse (2)

| Ingredients | Weight ratio (%) |
|---|---|
| Olive oil | 0.3 |
| Stearyl alcohol | 2.0 |
| Behenyl alcohol | 2.0 |
| Glycerin | 4.0 |
| 1,3-butylene glycol | 5.0 |
| Hydrolyzed wheat | 0.2 |
| Hydroxystearic acid | 0.5 |
| 2-ethylhexanoic acid cetyl | 1.0 |
| Hydroxypropyltrimonium chloride starch | 0.5 |
| Dimethylaminopropyl amide stearate | 0.5 |
| Test preparation (sample 2) | 1.0 |
| L-glutamic acid | 0.5 |
| Copolymer of aminoethylaminopropylsiloxane and dimethylsiloxane | 2.0 |
| Dimethylpolysiloxane | 0.5 |
| Phenoxyethanol | 0.5 |
| Pigment | As appropriate |
| Perfume | As appropriate |
| Purified water | The rest |

To purified water in Table 17 were added the cation-modified galactomannan polysaccharide of the present invention, hydroxypropyltrimonium chloride starch, dimethylaminopropyl amide stearate, hydrolyzed wheat, L-glutamic acid, glycerin, 1,3-butylene glycol, phenoxyethanol and pigment, and the mixture was maintained at 80°C (water phase). The remaining ingredients were mixed and heated so that they were dissolved, and the mixture was maintained at 80°C (oil phase). The water phase was combined with the oil phase, and the mixture was spun with a homogenizer to be emulsified. Then, the emulsion was cooled with stirring.

TABLE 18. Composition 6. Conditioner

| Ingredients | Weight ratio (%) |
|---|---|
| Cetanol | 1.5 |
| Behenyl alcohol | 4.0 |
| Glycerin | 3.0 |
| 2-ethylhexanoic acid cetyl | 2.0 |
| Isocetyl myristate | 0.5 |
| Polyoxyethylene(4)stearylether | 1.0 |
| N-(3-alkyl(12, 14)oxy-2-hydroxypropyl)-L-alginic hydrochloride | 0.6 |
| Test preparation (sample 5) | 0.6 |
| Behenic acid dimethylaminopropylamide | 1.5 |
| 50% Lactic acid | 0.9 |
| Phenoxy ethanol | 0.3 |
| Sodium benzoate | 0.3 |

Table continued

| Ingredients | Weight ratio (%) |
|---|---|
| Perfume | 0.5 |
| Purified water | The rest |

To purified water in Table 18 were added cation-modified galactomannan polysaccharide of the present invention, N-(3-alkyl(12, 14)oxy-2-hydroxypropyl)-L-alginic hydrochloride, dimethylaminopropylamide stearate, 50% lactic acid, glycerin, phenoxyethanol and sodium benzoate, and the mixture was maintained at 70°C (water phase). The remaining ingredients were combined and heated so that they were dissolved, and the mixture was maintained at 70°C (oil phase). The water phase was combined with the oil phase, and the mixture was spun with a homogenizer to be emulsified. Then, the emulsion was cooled with stirring.

Hair Color

(Preparation)

Example 10

10-a

The cation-modified cation-modified galactomannan polysaccharide prepared in Example 1 and 3 and named Samples 1 and 5 were used to prepare two-component type oxidation hair color samples whose composition was as shown in column (A) of Table 19 below. The two-component type oxidation hair color samples containing Samples 1 and 5 were named test prescriptions 16 and 17, respectively. At the time of use, the first and second components of each hair color sample was mixed at 1:1 weight ratio, and the mixture was applied to the hair.

TABLE 19. Prescription of Two-component type Oxidation Hair Color

<table>
<tr><th colspan="3">Weight ratio (%, in terms of solids) / Ingredients</th><th>(A)</th><th>Reference prescription (B)</th><th>Comparative prescription (C)</th></tr>
<tr><td rowspan="13">1st component</td><td>(1)</td><td>Test preparations (samples 1, 5)</td><td>0.5</td><td>0</td><td>0</td></tr>
<tr><td>(2)</td><td>Comparative preparation (Sample 8)</td><td>0</td><td>0</td><td>0.5</td></tr>
<tr><td>(3)</td><td>Cetostearylalcohol</td><td colspan="3">5.0</td></tr>
<tr><td>(4)</td><td>POE(5)behenylether</td><td colspan="3">2.0</td></tr>
<tr><td>(5)</td><td>POE(20)stearylether</td><td colspan="3">2.0</td></tr>
<tr><td>(6)</td><td>Highly polymerized silicone</td><td colspan="3">0.2</td></tr>
<tr><td>(7)</td><td>Propylene glycol</td><td colspan="3">2.0</td></tr>
<tr><td>(8)</td><td>Liquid paraffin</td><td colspan="3">5.0</td></tr>
<tr><td>(9)</td><td>28% aqueous ammonium solution</td><td colspan="3">2.0</td></tr>
<tr><td>(10)</td><td>Toluene-2,5-diamine</td><td colspan="3">1.5</td></tr>
<tr><td>(11)</td><td>Resorcin</td><td colspan="3">1.0</td></tr>
<tr><td>(12)</td><td>Disodium edetate</td><td colspan="3">0.2</td></tr>
<tr><td>(13)</td><td>Purified water</td><td colspan="3">The rest</td></tr>
<tr><td rowspan="9">2nd component</td><td>(1)</td><td>Aqueous hydrogenperoxide solution (35%)</td><td colspan="3">17.0</td></tr>
<tr><td>(2)</td><td>Cetanol</td><td colspan="3">2.0</td></tr>
<tr><td>(3)</td><td>POE(30)cetylether</td><td colspan="3">3.0</td></tr>
<tr><td>(4)</td><td>POE(2)laurylether sulfuric acid sodium</td><td colspan="3">0.3</td></tr>
<tr><td>(5)</td><td>Methylparaben</td><td colspan="3">0.1</td></tr>
<tr><td>(6)</td><td>Citric acid (pH 3.5)</td><td colspan="3">As appropriate</td></tr>
<tr><td>(7)</td><td>Disodium hydrogenphosphate</td><td colspan="3">0.1</td></tr>
<tr><td>(8)</td><td>Disodium edetate</td><td colspan="3">0.2</td></tr>
<tr><td>(9)</td><td>Purified water</td><td colspan="3">The rest</td></tr>
</table>

## 10-b (Preparation of comparative prescriptions)

To compare the effects of the cation-modified galactomannan polysaccharide of the present invention in hair color preparations, a comparative hair color sample was prepared by using the cation-modified galactomannan polysaccharide obtained in Comparative Example 1 in which the composition ratio of mannose as a main chain and galactose as a side chain is different from that of the present invention, i.e., sample 8 in Table 1, to which ingredients specified under column (C) of Table 19 were used to provide a two-component type oxidation hair color sample. The two-component type oxidation hair color sample which contained cation-modified galactomannan polysaccharide of sample 8 in Table 1 was named comparative prescription 10. At the time of use, the first and second components of the hair color sample was mixed at 1:1 weight ratio, and the mixture was applied to the hair.

(Evaluation)

Example 11

In this test, test prescriptions 16 and 17 prepared as described in 10-a, and a reference prescription were used. The reference prescription had a composition as shown in column (B) of Table 19. Preparation of the reference prescription occurred as in the test prescriptions, except that it did not contain any cationic polymer.

For each of the test prescriptions, the first and second components were combined in equal amounts prior to use, and the mixture was applied to the hair. The hair was left at room temperature for 30 minutes, rinsed for 3 minutes with running water kept at 40°C, and dried with a drier.

Ten testers participated to evaluate the performance of the test prescriptions as compared with the reference prescription (B) with special attention being paid to:

* smoothness of the hairs during rinsing, and
* feel (moist feeling, flexibility) after dry. The tester's evaluation was scored according to the following rank scale.

For each of the test prescriptions, the scores of the ten testers were summed and the sum was taken as its overall evaluation result (in Table 20). The evaluation results of the test prescriptions are shown in Table 21. The above-mentioned reference prescription (B) was prepared according to the above 10-a, and the first and second components of each of hair color samples were mixed at 1:1 wieght ratio, and the mixture was applied to hair.

Comparative Example 4

With regard to comparative prescription 10 prepared as described in 10-b, its performance was compared with that of the reference prescription in the same manner as in Example 11. The thus obtained evaluation results are shown in Table 21.

TABLE 20. Ranking Scale for Evaluating Test Prescriptions

| | Test properties | |
|---|---|---|
| Score | Smoothness during rinsing | Finished feel after drying hair |
| +2 | Better | Better |
| +1 | Slightly better | Slightly better |
| 0 | The same | The same |
| -1 | Slightly worse | Slightly worse |
| -2 | Worse | Worse |

TABLE 21. Evaluation Results of Hair Color Prescriptions

| Test cationic polymers | | Test prescriptions | | Comparative prescriptions |
|---|---|---|---|---|
| | | 16 | 17 | 10 |
| | | Sample 1 | Sample 5 | Sample 8 |
| Smoothness during rinsing | | 14 | 13 | 10 |
| Feel after drying hair | Moist feeling | 15 | 14 | 4 |
| | Flexibility | 15 | 16 | 5 |

The results of Table 21 indicate that the cation-modified galactomannan polysaccharide of the present invention, when added to a hair color preparation, enhances the smoothness of the hairs during rinsing which will contribute to the treatment of damaged hairs, and enables easy finger passing. It also improves the moist feeling of the hairs after drying. It was demonstrated that the cation-modified cation-modified galactomannan polysaccharide of the invention brings about beneficial effects when added to a hair color composition.

When the test prescriptions are compared with comparative prescription 10, the former preparations 16 and 17 of the present invention ensure more improved feel after drying hair and treat more effectively damaged hairs in comparison with the comparative prescription 10 which contained the sample 8 and was the same galactomannan polysaccharide, but prepared by cation-modifying gar gum in which the composition ratio of mannose and glactose is different.

(Preparation Examples 7 and 8)

Preferred hair color compositions according to the invention which are effective in treating damaged hairs and can exert conditioning effects on the hairs and enhance elasticity of the hairs will be described below.

TABLE 22. Preparation Example 7. Prescription of Two-Components type Oxidation Hair Color

| Ingredients | | | Weight ratio (%) |
|---|---|---|---|
| 1st component | (1) | Test preparations (samples 2) | 0.3 |
| | (2) | Polyquaternium-6 | 0.3 |
| | (3) | Stearyltrimethylammonium chloride | 1.0 |
| | (4) | POE(20)oleylether | 5.0 |
| | (5) | POE(20)hexyldecylether | 10.0 |
| | (6) | Liquid amidopropylbetaine laurate | 5.0 |
| | (7) | Cetanol | 5.0 |
| | (8) | Propylene glycol | 15.0 |
| | (9) | Polyethylene glycol | 5.0 |
| | (10) | Sodium thioglycolate | 0.5 |
| | (11) | Aminopropyldimethicone | 0.4 |
| | (12) | Toluene 2,5-diamine | 3.0 |
| | (13) | Resorcin | 0.8 |
| | (14) | 2,4-diaminophenoxyethanol hydrochloric acid | 0.2 |
| | (15) | Paraphenylene diamine | 0.2 |
| | (16) | Metha-aminophenol | 0.2 |
| | (17) | 28% aqueous ammonium solution | 5.0 |
| | (18) | Sodium sulfite | 0.5 |
| | (19) | Perfume | As appropriate |
| | (20) | Preservative | As appropriate |
| | (21) | Purified water | The rest |
| 2nd component | (1) | Aqueous hydrogenperoxide solution (35%) | 17.0 |
| | (2) | Cetanol | 1.0 |
| | (3) | Sodium larylsulfate | 0.3 |
| | (4) | Phenacetin | 0.1 |
| | (5) | Disodium edetate | 0.2 |
| | (6) | Purified water | The rest |

TABLE 23. Preparation Example 8. Prescription of Two-component type Oxidation Hair Color

| Ingredients | | | Weight ratio (%) |
|---|---|---|---|
| 1st component | (1) | Test preparations (samples 5) | 1.0 |
| | (2) | Aqueous solution of hydrolyzed collagen (36.5%) | 3.0 |
| | (3) | Coconut oil fatty acid amide propyldimethylaminoacetic acid betaine | 30.0 |
| | (4) | POE(2)dodecylether | 15.0 |
| | (5) | POE(10)dodecylether | 15.0 |
| | (6) | 2-octyldecanol | 5.0 |
| | (7) | Polyethylene glycol | 5.0 |
| | (8) | Trimethylbehenylammonium chloride | 1.0 |
| | (9) | 28% aqueous ammonium solution | 2.0 |
| | (10) | Ascorbic acid | 0.5 |
| | (11) | Anhydrous sodium sulfite | 0.2 |
| | (12) | Perfume | As appropriate |
| | (13) | Preservative | As appropriate |
| | (14) | Purified water | The rest |
| 2nd component | (1) | Aqueous hydrogenperoxide solution (35%) | 15.0 |
| | (2) | Cetanol | 2.0 |
| | (3) | POE(20)cetylether | 3.0 |
| | (4) | Sodium larylsulfate | 0.5 |
| | (5) | Methylparaben | 0.1 |
| | (6) | glycolic acid | As appropriate |
| | (7) | Disodium hydrogenphosphate | 0.1 |
| | (8) | Disodium edetate | 0.2 |
| | (9) | Purified water | The rest |

Body Cleansing Preparation

(Preparation)

Example 12

12-a

The cation-modified galactomannan polysacchardie prepared in Examples 1 and 2, and named Samples 1, 2 and 4 were used to prepare body cleansing samples (body soaps) whose composition was as shown in column (A) of Table 24 below. Specifically, ingredient (11) was heated to 60°C, to which ingredient (1) was added with stirring and dissolved. After the dissolution was recognized, ingredients (3) to (7) were added to the solution with stirring and the mixture was further stirred until it became a uniform solution. Further, ingredients (8) to (10) were added to the mixture at 30 to 40°C with stirring in the same manner as above until they were dissolved to homogeneity. Thus, body cleansing samples with compositions as shown in column (A) of Table 24 were obtained. The three body cleansing samples containing the cation-modified galactomannan polysaccharide represented by samples 1, 2 and 4 were named test prescriptions 18, 19 and 20, respectively.

TABLE 24. Prescription of Body Cleansers (Body Soaps)

| Ingredients / Weight ratio (%, in terms of solids) | | (A) | Reference prescription (B) | Comparative prescriptions (C) |
|---|---|---|---|---|
| (1) | Test samples (sample 1, 2, 4) | 0.4 | 0 | 0 |
| (2) | Comparative samples (sample 8, fenugreek gum, Cational HC-100) | 0 | 0 | 0.4 |
| (3) | Lauryl sulfuric acid ammonium | 9.5 | 9.5 | 9.5 |
| (4) | Polyoxyethylene(3)laurylether sulfuric acid sodium | 2 | 2 | 2 |
| (5) | Polyoxyethylene(2)laurylether phosphoric acid sodium | 4 | 4 | 4 |
| (6) | Coconut oil fatty acid amide propylbetaine | 3 | 3 | 3 |
| (7) | Coconut oil monoethanol amide | 2.7 | 2.7 | 2.7 |
| (8) | Disodium edetate | 0.2 | 0.2 | 0.2 |
| (9) | Sodium benzoate | 0.2 | 0.2 | 0.2 |
| (10) | Aqueous solution of citric acid (for pH adjustment, pH 5.7-6.2) | As appropriate | As appropriate | As appropriate |
| (11) | Distilled water | The rest | The rest | The rest |

12-b (Preparation comparative prescription)

To compare the effects of the cation-modified galactomannan polysaccharide of the present invention in body cleansing preparations, a comparative body cleansing sample was prepared by using fenugreek gum which was an unmodified or intact galactomannan polysaccharide, to which ingredients specified under column (C) of Table 24 were added to provide a body cleansing sample. Specifically, ingredient (11) was heated to 60°C, to which ingredient (2) was slowly added with stirring and dissolved. After the dissolution was recognized, ingredients (3) to (7) were added to the solution at 50 to 60°C with stirring and the mixture was further stirred until it became a uniform solution. Further, ingredients (8) to (10) were added to the solution at 30 to 40°C with stirring in the same manner as above, until they were dissolved to homogeneity. Thus, a body cleansing sample with a composition as shown in column (C) of Table 24 was obtained. The sample was named comparative prescription 11. 12-c (Preparation of comparative prescription)

In the same manner as above, to compare the effects of the cation-modified cation-modified galactomannan polysaccharide of the present invention with those of a conventional cationic polymer in body cleansing preparations, another comparative body cleansing samples having composition shown in (C) of Table 24 as other cationic polymer were prepared by using a cation-modified hydroxyethyl cellulose containing nitrogen at 1.8 wt% into which an average of 1.8 mol of ethylene oxide was introduced (Catinal HC-100, Toho Chemical Industry), and the Sample 8 obtained in the Comparative Example 1. Specifically, ingredient (11) was heated to 60°C, to which ingredient (2) was slowly added with stirring and dissolved. After recognizing the dissolution, ingredients (3) to (7) were added to the solution at 50 to 60°C with stirring and the mixture was further stirred until it became a uniform solution. Then, ingredients (8) to (10) were added to the solution at 30 to 40°C with stirring in the same manner as above, until they were dissolved to homogeneity. Thus, body cleansing samples with a composition as shown in column (C) of Table 24 were obtained. The samples were named comparative prescriptions 12 and 13.

(Evaluation)

Example 13

In this test, with respect to the test prescriptions 18, 19 and 20 prepared as described in 12-a, performance evaluation shown in the below-mentioned items was conducted by ten testers. The formance evaluation method used a body cleansing sample having composition shown by a reference prescription (B) of Table 24 which does not contain high polymer compound such as cationic polymer, etc. as ingredient, and body cleansing samples to be evaluated.

The testers evaluated the performance of the test prescriptions as compared with the reference prescription (B) with use feel being paid to:

* foam amount and foam quality during use,
* use feel during use (easy removal after rinsing, no tautness and sliminess after rinsing), and
* use feel of the dried skin after use (no tautness, smoothness, and moist feeling). The tester's evaluation was scored according to the following rank scale.

For each of the test prescriptions, the scores of the ten testers were summed in Tables 25 and 26 and the sum was taken as its overall evaluation result by the system sthon in Table 27.

The reference prescription was prepared in such a manner that ingredient (11) of the reference prescription (B) in the Table 24 was heated to 60°C, to which ingredients (3) to (7) were added at 50 to 60°C with stirring and dissolved to homogeneity, and further, ingredients (8) to (10) were added to the solution at 30 to 40°C with stirring similarly and the mixture was further stirred it became a uniform solution. Thus, a body cleansing sample with a composition as shown in column (B) of Table 24 was obtained. The sample was used as the reference prescription in this test.

Comparative Example 5

With regard to comparative prescriptions 11, 12 and 13 prepared as described in 12-b and 12-c, their performance was compared with that of the reference prescription in the same manner as in Example 10. The thus obtained evaluation results are shown in Table 27.

TABLE 25. Ranking Scale for Evaluating Test Prescriptions (During Use)

| | Test properties | | |
|---|---|---|---|
| Score | Foam amount | Texture of foam (foam quality) | |
| +2 | Higher | Better | |
| +1 | Slightly higher | Slightly better | |
| 0 | The same | The same | |
| -1 | Slightly lower | Slightly worse | |
| -2 | lower | Worse | |
| | Test properties | | |
| Score | Rinsing easiness | Tautness after rinsing | Sliminess after rinsing |
| +2 | Easier | Less taut | Less slimy |
| +1 | Slightly easier | Slightly less taut | Slightly less slimy |
| 0 | The same | The same | The same |
| -1 | Slightly harder | Slightly more taut | Slightly more |
| -2 | Harder | More taut | More slimy |

TABLE 26. Ranking Scale for Evaluating Test Prescriptions (After Use)

| | Test properties | | |
|---|---|---|---|
| Score | Tautness after dried | Smoothness after dried | Moist feeling after dried |
| +2 | Less taut | Smoother | More moisture-rich |
| +1 | Slightly less taut | Slightly smoother | Slightly more moisture-rich |
| 0 | The same | The same | The same |
| -1 | Slightly more taut | Slightly more | Slightly less moisture-rich |
| -2 | More taut | More coarse | Less moisture-rich |

The results of Table 27 indicate that the inventive cation-modified galactomannan polysaccharide, when added to a body cleansing preparation, improves lathering, foam quality and use feel of the body cleansing preparation, does not remove oil and fat component of skin, and causes no tautness in the skin and enhances the moist feeling of the skin after use, due to the emulsifiability of the cation-modified galactomannan polysaccharide of the present invention.

When the test prescriptions are compared with comparative prescription 11 containing an unmodified or intact cation-modified galactomannan polysaccharide (fenugreek gum), it was recognized that the former are found to be adsorbed more readily to the skin because of cation-modification, and improve feel during and after use. When the test prescriptions are compared with another comparative sample (comparative prescription 12) containing cation-modified hydroxyethyl cellulose or cation-modified guar gum (comparative prescription 13) prepared by cation-modifying guar gum which is the same galactomannan polysaccharide, but is poor in emulsifiability, the former not only relieve the skin of sliminess, but also relieve the skin of chapping and tautness by appropriately retaining oil moiety due to the difference of emulsifiability.

TABLE 27. Evaluation Results of Prescriptions of Ceansing Body

| | | Test prescriptions | | | Comparative prescriptions | | |
|---|---|---|---|---|---|---|---|
| | | 18 | 19 | 20 | 11 | 12 | 13 |
| Test cationic polymers | | Sample 1 | Sample 2 | Sample 4 | Intact cation-modified galacto-mannan poly-saccharide (fenugreek gum) | Cation-modified hydroxy-cellulose (Cational HC-100) | Sample 8 |
| During use | Foam amount | 16 | 15 | 17 | -3 | 12 | 11 |
| | Foam quality | 12 | 13 | 13 | -1 | 10 | 11 |
| | Rinsing easiness | 8 | 7 | 6 | -6 | -2 | 3 |
| | Tautness after rinsing | 11 | 10 | 10 | 2 | 8 | 7 |
| | Sliminess after rinsing | 10 | 9 | 7 | -1 | -6 | 2 |
| After use | Tautness after dried | 11 | 12 | 11 | -6 | -1 | 3 |
| | Smoothness after dried | 9 | 9 | 7 | 0 | -3 | -1 |
| | Moist feeling after dried | 10 | 11 | 11 | 1 | 5 | 4 |

(Preparation Examples 9-15)

Preferred skin treatment compositions such as body cleansers, etc., according to the invention which are effective in improving the feel owing to conditioning effects and emulsifiability of the present invention will be described below.

TABLE 28. Preparation Example 9. Bodv Cleanser (1)

| Ingredients | Weight ratio (%) |
|---|---|
| Propylene glycol | 3.0 |

Table continued

| Ingredients | Weight ratio (%) |
|---|---|
| Polyoxypropylene glycerylether | 1.0 |
| Stearic acid | 1.0 |
| Lauric acid | 1.0 |
| Palm fatty acid | 10.0 |
| Myristic acid | 10.0 |
| Ethylene glycol distearate | 1.0 |
| Coconut oil fatty acid diethanolamide | 1.0 |
| Polyoxyethylene(2)laurylether sulfuric acid sodium | 5.0 |
| Coconut oil fatty acid taurine sodium | 2.0 |
| Aqueous solution of potassium hydroxide (47%) | 10.0 |
| Test preparation (sample 2) | 0.4 |
| Disodium edetate | 0.1 |
| Sodium benzoate | 0.1 |
| Purified water | The rest |
| Perfume | As appropriate |

The above ingredients were combined according to the conventional method to produce a liquid body cleansing preparation (body soap).

TABLE 29. Preparation Example 10. Body Cleanser (2)

| Ingredients | Weight ratio (%) |
|---|---|
| Polyoxyethylene(2)laurylether phosphate | 12.0 |
| Polyoxyethylene(3)laurylether sulfuric acid sodium | 3.0 |
| Coconut oil sarcosine triethanolamine | 1.0 |
| Coconut oil fatty acid diethanolamide | 1.0 |
| Ethyleneglycol distearate | 1.5 |
| Coconut oil fatty acid amide propylbetaine | 3.0 |
| Dipropylene glycol | 4.0 |
| Coconut oil fatty acid taurine sodium | 1.0 |
| Triethanolamine | 4.3 |
| Test preparation (sample 1) | 0.2 |
| Hydroxypropylmethyl cellulose | 0.05 |
| Glycerin | 0.1 |
| Disodium edetate | As appropriate |
| Preservative, Perfume | As appropriate |
| Purified water | The rest |

The above ingredients were combined according to the conventional method to produce a liquid body cleansing preparation (body soap).

TABLE 30. Preparation Example 11. After-shave lotion

| Ingredients | Weight ratio (%) |
|---|---|
| Carboxyvinyl polymer | 0.3 |
| Isodecyl oleate | 8.0 |
| 2-hexyldecyl palmitate | 3.0 |
| Polyoxyethylenecetylether(18) | 3.0 |
| Octyl dodecanol | 4.0 |
| Glycerin | 3.0 |
| Cetanol | 1.0 |
| Ethanol | 3.0 |
| Test preparation (sample 3) | 1.0 |
| Paraoxybenzoic acid methyl | 0.2 |
| Squalane | 0.2 |
| Hydrolyzed silk | 0.2 |
| Menthol | As appropriate |
| Pigment | As appropriate |
| Perfume | As appropriate |
| 25% aqueous solution of sodium hydroxide (for pH adjustment, pH5.7-6.2) | As appropriate |
| Distilled water | The rest |

The above ingredients were combined by conventional method to produce liquid after shave lotion.

TABLE 31. Composition 12. Bath preparation

| Ingredients | Weight ratio (%) |
|---|---|
| Magnesium palmitate | 2.0 |
| Calcium stearate | 3.0 |
| Liquid paraffin | 9.0 |
| Cetanol | 2.0 |
| Carboxyvinyl polymer | 0.15 |
| Triethanolamine | 0.13 |
| Test preparation (sample 5) | 0.4 |
| Squalane | 2.0 |
| Perfume | As appropriate |
| Paraoxybenzoic acid methyl | 0.2 |
| Distilled water | The rest |

The above ingredients were combined according to the conventional method to produce a bath preparation.

(Preparation Examples 13-14)

Other example of application-type hair treatment compositions which utilizes conditioning effect of the cation-modified galactomannan polysaccharide of the present invention to keratin will be described below.

TABLE 32. Preparation Example 13. Hair Gel

| Ingredients | Weight ratio (%) |
|---|---|
| Test preparation (sample 2) | 2.0 |
| Glycerin | 5.0 |
| Ethanol | 20.0 |
| Polyoxyethyleneoctyldecylether | 1.0 |
| Perfume, Chelating agent | As appropriate |
| Purified water | The rest |

One part of the specified amount of purified water was added to the present invention and glycerin, and the mixture was heated to 70°C and dissolved. Other ingredients were dissolved in the remaing part of the purified water and added to the above solution with stirring.

TABLE 33. Preparation Example 14. Hair Mousse

| Ingredients | Weight ratio (%) |
|---|---|
| Methylpolysiloxane | 2.0 |
| Copolymer of polyoxyethylene and methylpolysiloxane | 0.3 |
| Test preparation (sample 1) | 2.0 |
| Ethanol | 4.0 |
| Cetanol | 0.5 |
| Propylene glycol | 1.0 |
| Dried beewax | 1.0 |
| Stearic acid | 0.5 |
| Myristic acid | 1.0 |
| Palmitic acid | 5.0 |
| Polyoxyethylenesorbitan monostearate (20E.O.) | 5.0 |
| Polyoxyethylene hardened caster oil | 1.0 |
| Polyoxyethylenecetyl ether | 0.5 |
| Triethanol amine | 1.2 |
| Eucalyptus oil | As appropriate |
| L-menthol | As appropriate |
| Phenoxyethanol | 0.2 |
| Mixture of polyurethane and polyether modified silicone | 15.0 |
| Purified water | The rest |
| Liquefied petroleum gas | 12.0 |

TABLE 34. Preparation Example 15. Hair Wax

| Ingredients | Weight ratio (%) |
|---|---|
| Liquid paraffin | 10.0 |
| Micro-crystalline wax | 10.0 |

Table continued

| Ingredients | Weight ratio (%) |
|---|---|
| Methylpolysiloxane | 5.0 |
| Stearylalcohol | 3.0 |
| Propylene glycol | 10.0 |
| Carnauba wax | 3.0 |
| Isostearic acid | 1.0 |
| Stearic acid | 5.0 |
| Test preparation (sample 2) | 1.0 |
| Tetra 2-ethylenehexanic acid pentaelisulit | 2.0 |
| Polyoxyethylene hardened castor oil | 3.0 |
| Polyoxyethylenelaurylether phosphoric acid | 2.0 |
| Self-emulsified monostearic acid glyceryl | 2.0 |
| Triethanolamine | 1.0 |
| D- δ -tocophenol | 0.05 |
| Oat extract | 0.1 |
| Paraoxybenzoic acid ester | 0.2 |
| Polyacrylic acid sodium | 0.05 |
| Purified water | The rest |
| Perfume | 0.08 |

The cation-modified galactomannan polysaccharide of the present invention, when it is used as a constituent of a hair treatment composition such as shampoo, hair rinse, etc., it exerts conditioning effects on the hair during use, as good as or better than cationic polymers which have been used as a conventional hair conditioning agent, enhances moisture-richness and flexibility after drying as a result of the emulsifiability. The cation-modified galactomannan polysaccharide of the present invention, when added to a topical skin care composition such as body cleansers, etc., it relieves the skin of sliminess and tautness, and enhances its moist feeling. Accordingly, it is possible to provide more useful cosmetic compositions than corresponding conventional products.

## Claims

1. A cation-modified galactomannan polysaccharide which is cation-modified polysaccharide obtained by substituting some of hydroxyl groups contained in a galactomannan polysaccharide which is nonionic polysaccharide which constitutes mannose as a main chain and galactose as a side chain and the composition ratio of mannose and galactose is 1:1, with quaternary nitrogen-containing groups of the following Formula (1), and having the cationic charge density due to the quaternary nitrogen-containing group being 0.1 to 3.0 meq/g:

[Chemical Formula 1]

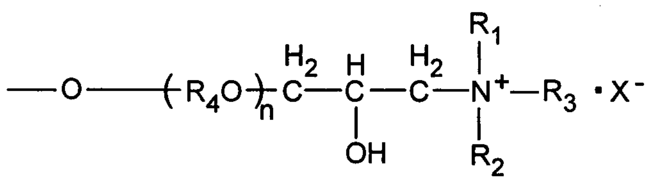

wherein $R_1$ and $R_2$ independently represent C1 to 3 alkyl groups; $R_3$ represents C1 to 24 alkyl group; X- represents

an anion; and n is 0 or an integer of 1-30, and when n=1-30, $(R_4O)_n$ represents a residue of C2 to 4 alkyleneoxide polymer, that is, a polyalkylene glycol chain comprising unialkyleneoxide and/or two or more different alkyleneoxides.

**2.** A cation-modified galactomannan polysaccharide according to Claim 1, wherein the galactomannan polysaccharide in which the composition ratio of mannose and galacose is 1:1 is a water-soluble natural gum obtained from albumen portion of a seed of fenugreek (Trigonella foenum-graecum) which is a leguminus plant.

**3.** A cation-modified cation-modified galactomannan polysaccharide according to Claims 1 and 2, wherein cation-modification of the cation-modified galactomannan polysaccharide is achieved by using a glycidyltrialkyl ammonium salt or a 3-halogeno-2-hydroxypropyltrialkyl ammonium salt.

**4.** A cation-modified cation-modified galactomannan polysaccharide according to Claims 1 to 3, wherein cation-modification of the cation-modified galactomannan polysaccharide is made by adding C2-4 alkyleneoxide to some of hydroxyl groups contained in said cation-modified galactomannan polysaccharide, and then using glycidyltrialkyl ammonium salt or 3-halogeno-2-hydroxyproplyltrialkyl ammonium salt which acts as a cation-modification agent.

**5.** A cosmetic composition containing the cation-modified galactomannan polysaccharide as described in Claims 1 to 4.

**6.** A cosmetic composition according to Claim 5, wherein the content of the cation-modified galactomannan polysaccharide as described in Claims 1 to 4 is 0.05 to 5 % by weight with respect to the weight (100% by weight) of the composition.

**7.** A cosmetic composition **characterized by** containing an additional cationic water-soluble polymer and/or amphoteric water-soluble polymer at 5 % by weight or lower with respect to the weight (100% by weight) of the composition, in the cosmetic composition described in Claims 5 and 6.

**8.** A cosmetic composition **characterized by** containing an amideamine compound and a neutralizing agent such as an organic and/or inorganic acid, a higher fatty acid and/or higher alcohol, in the cosmetic composition described in Claims 5 to 7.

**9.** A cosmetic composition according to Claims 5 to 8 which is a hair treatment composition.

**Amended claims under Art. 19.1 PCT**

**1.** (After amendment) A cation-modified galactomannan polysaccharide which is cation-modified polysaccharide obtained by substituting some of hydroxyl groups contained in a galactomannan polysaccharide which is nonionic polysaccharide which constitutes mannose as a main chain and galactose as a side chain and the composition ratio of mannose and galactose is 1:1, with quaternary nitrogen-containing groups of the following Formula (1), and having the cationic charge density due to the quaternary nitrogen-containing group being 0.1 to 3.0 meq/g:

[Chemical Formula 1]

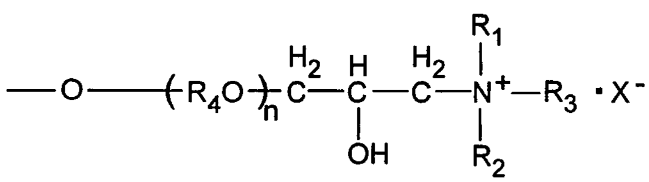

wherein $R_1$ and $R_2$ independently represent C1 to 3 alkyl groups; $R_3$ represents C1 to 24 alkyl group; $X^-$ represents an anion; and n is 0 or an integer of 1-30, and when n=1-30, $(R_4O)_n$ represents a residue of C2 to 4 alkyleneoxide polymer, that is, a polyalkylene glycol chain comprising unialkyleneoxide and/or two or more different alkyleneoxides, with the exception of a case that n=0 and $R_1$, $R_2$ and $R_3$ are simultaenously methyl group.

**2.** A cation-modified galactomannan polysaccharide according to Claim 1, wherein the galactomannan polysaccharide in which the composition ratio of mannose and galacose is 1:1 is a water-soluble natural gum obtained from

albumen portion of a seed of fenugreek (Trigonella foenum-graecum) which is a leguminus plant.

**3.** (After amendment) A cation-modified cation-modified galactomannan polysaccharide according to Claims 1 and 2, wherein cation-modification of the cation-modified galactomannan polysaccharide is achieved by using a glycidyltrialkyl ammonium salt or a 3-halogeno-2-hydroxypropyltrialkyl ammonium salt, with the exception of N-(3-chloro-2-hydroxyproply)trimethyl ammonium salt.

**4.** (After amendment) A cation-modified cation-modified galactomannan polysaccharide according to Claim 1 or 2, wherein cation-modification of the cation-modified galactomannan polysaccharide is made by adding C2-4 alkyleneoxide to some of hydroxyl groups contained in said cation-modified galactomannan polysaccharide, and then using glycidyltrialkyl ammonium salt or 3-halogeno-2-hydroxyproplyltrialkyl ammonium salt which acts as a cation-modification agent.

**5.** (After amendment) A cosmetic composition containing a cation-modified galactomannan polysaccharide which is cation-modified polysaccharide obtained by substituting some of hydroxyl groups contained in a galactomannan polysaccharide which is nonionic polysaccharide which constitutes mannose as a main chain and galactose as a side chain and the composition ratio of mannose and galactose is 1:1, with quaternary nitrogen-containing groups of the following Formula (1), and having the cationic charge density due to the quaternary nitrogen-containing group being 0.1 to 3.0 meq/g:

[Chemical Formula 1]

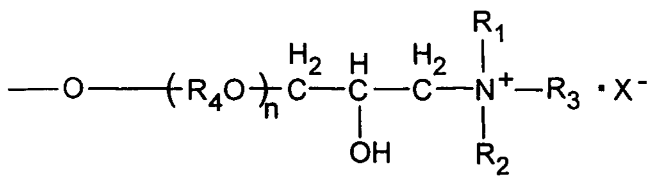

wherein $R_1$ and $R_2$ independently represent C1 to 3 alkyl groups; $R_3$ represents C1 to 24 alkyl group; X- represents an anion; and n is 0 or an integer of 1-30, and when n=1-30, $(R_4O)_n$ represents a residue of C2 to 4 alkyleneoxide polymer, that is, a polyalkylene glycol chain comprising unialkyleneoxide and/or two or more different alkyleneoxides, provided that a case that n=0 and $R_1$, $R_2$ and $R_3$ together represent methyl group.

**6.** (Addition) A cosmetic composition according to Claim 5, wherein the galactomannan polysaccharide in which the composition ratio of mannose and galacose is 1:1 is a water-soluble natural gum obtained from albumen portion of a seed of fenugreek (Trigonella foenum-graecum) which is a leguminus plant.

**7.** (Addition) A cosmetic composition according to Claim 5 or 6, wherein cation-modification of the cation-modified galactomannan polysaccharide is achieved by using a glycidyltrialkyl ammonium salt or a 3-halogeno-2-hydroxypropyltrialkyl ammonium salt.

**8.** (Addition) A cosmetic composition according to Claim 5 or 6, wherein cation-modification of the cation-modified galactomannan polysaccharide is made by adding C2-4 alkyleneoxide to some of hydroxyl groups contained in said cation-modified galactomannan polysaccharide, and then using glycidyltrialkyl ammonium salt or 3-halogeno-2-hydroxyproplyltrialkyl ammonium salt which acts as a cation-modification agent.

**9.** (After amendment) A cosmetic composition according to Claim 5, wherein the content of the cation-modified galactomannan polysaccharide as described in Claims 5 to 9 is 0.05 to 5 % by weight with respect to the weight (100% by weight) of the composition.

**10.** (After amendment) A cosmetic composition **characterized by** containing an additional cationic water-soluble polymer and/or amphoteric water-soluble polymer at 5 % by weight or lower with respect to the weight (100% by weight) of the composition, in the cosmetic composition described in Claims 5 to 9.

**11.** (After amendment) A cosmetic composition **characterized by** containing an amideamine compound and a

neutralizing agent such as an organic and/or inorganic acid, a higher fatty acid and/or higher alcohol, in the cosmetic composition described in Claims 5 to 10.

**12.** (After amendment) A cosmetic composition according to Claims 5 to 8 which is a hair treatment composition.

**Statement under Art. 19.1 PCT**

Amendments to the claims 1 and 3 were made to clarify the difference from the invention described by WO 00/31339 A1 raised in the International Search Report. Namely, with respect to quaternary nitrogen-containing group represented by Formula (1), limitation of the exception of a case that n=0 and $R_1$, $R_2$ and $R_3$ are not simultaneously methyl group is added to the claim 1, and with respect to 3-halogeno-2-hydroxypropyltrialkyl ammonium salt, limitation of the exception of N-(3-chloro-2-hydroxypropyl)trimethyl ammonium salt is added to the claim 3. Furthermore, claims 6, 7 and 8 are newly added and thus, the claim numbers of the original claims 6 to 9, i.e., before the amendment, are renumbered to be claims 9 to 12, respectively.

**INTERNATIONAL SEARCH REPORT**

International application No.
PCT/JP2004/006512

A. CLASSIFICATION OF SUBJECT MATTER
Int.Cl[7] C08B37/00, A61K7/06, 7/075, 7/08, 7/13, 7/50

According to International Patent Classification (IPC) or to both national classification and IPC

B. FIELDS SEARCHED

Minimum documentation searched (classification system followed by classification symbols)
Int.Cl[7] C08B37/00, A61K7/06, 7/075, 7/08, 7/13, 7/50

Documentation searched other than minimum documentation to the extent that such documents are included in the fields searched

Electronic data base consulted during the international search (name of data base and, where practicable, search terms used)
CA(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 2002-530549 A (Hercules Inc.),<br>17 September, 2002 (17.09.02),<br>Claim 3; Par. Nos. [0012], [0015]<br>& WO 00/31339 A1 | 1-4<br>5-9 |
| A | JP 2003-512152 A (Rodia Chimie),<br>02 April, 2003 (02.04.03),<br>Claim 9<br>& WO 01/028673 A1 | 1-9 |
| A | JP 2000-103724 A (Toho Chemical Industry Co., Ltd.),<br>11 April, 2000 (11.04.00),<br>Claim 1<br>(Family: none) | 1-9 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 22 July, 2004 (22.07.04) | 10 August, 2004 (10.08.04) |
| Name and mailing address of the ISA/<br>Japanese Patent Office | Authorized officer |
| Facsimile No. | Telephone No. |

Form PCT/ISA/210 (second sheet) (January 2004)

**INTERNATIONAL SEARCH REPORT**

International application No.
PCT/JP2004/006512

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-500512 A (Rodia Chimie), 18 January, 2000 (18.01.00), Claim 1 & WO 97/18263 A1 | 1-9 |
| A | JP 3-500302 A (DIAMALT AG.), 24 January, 1991 (24.01.91), Claim 2 & WO 89/02900 A1 | 1-9 |
| P,A | JP 2004-123569 A (Fancl Corp.), 22 April, 2004 (22.04.04), Claim 1 (Family: none) | 1-9 |
| P,A | JP 2003-327603 A (Toho Chemical Industry Co., Ltd.), 19 November, 2003 (19.11.03), Claim 1 (Family: none) | 1-9 |

Form PCT/ISA/210 (continuation of second sheet) (January 2004)